# EUROPÄISCHE PATENTANMELDUNG

(11) **EP 4 270 406 A1**
(43) Veröffentlichungstag der Anmeldung: **01.11.2023**
(21) Anmeldenummer: 22169831.9
(22) Anmeldetag: 25.04.2022
(51) Int. Cl.: G16H 20/13, G16H 40/63, G16H 40/20

(54) **VERFAHREN UND VORRICHTUNG ZUM VERARBEITEN VON MIT WENIGSTENS EINER MEDIKAMENTENAUSGABEVORRICHTUNG ASSOZIIERTEN DATEN**

(71) Anmelder: JDM Innovation GmbH, 71711 Murr (DE)
(72) Erfinder: KOCH, Daniel, 71686 Remseck (DE); LAFLEUR, Martin, 71711 Steinheim (DE); LUFT, Daniel, 70771 Leinfelden-Echterdingen (DE)
(74) Vertreter: DREISS Patentanwälte PartG mbB

(57) **Zusammenfassung**

Verfahren zum Verarbeiten von mit wenigstens einer Medikamentenausgabevorrichtung assoziierten Daten, aufweisend: Ermitteln von ersten Informationen, die zumindest Teile eines mit wenigstens einem Medikament assoziierten Medikationsplans charakterisieren, Bilden von zweiten Informationen für eine Steuerung der Medikamentenausgabevorrichtung basierend wenigstens auf den ersten Informationen, und, optional, Bereitstellen der zweiten Informationen, beispielsweise für eine Nutzung der zweiten Informationen durch die wenigstens eine Medikamentenausgabevorrichtung.

## Beschreibung

Die Offenbarung betrifft ein Verfahren zum Verarbeiten von mit wenigstens einer Medikamentenausgabevorrichtung assoziierten Daten.

Die Offenbarung betrifft ferner eine Vorrichtung zum Verarbeiten von mit wenigstens einer Medikamentenausgabevorrichtung assoziierten Daten.

Beispielhafte Ausführungsformen beziehen sich auf ein Verfahren, beispielsweise ein computerimplementiertes Verfahren, zum Verarbeiten von mit wenigstens einer Medikamentenausgabevorrichtung, beispielsweise zur Ausgabe von in Blisterbeuteln bzw. in einem Blisterbeutelschlauch angeordneten Medikamenten, assoziierten Daten, aufweisend: Ermitteln von ersten Informationen, die zumindest Teile eines mit wenigstens einem Medikament assoziierten Medikationsplans charakterisieren, Bilden von zweiten Informationen für eine Steuerung der wenigstens einen Medikamentenausgabevorrichtung basierend wenigstens auf den ersten Informationen, und, optional, Bereitstellen der zweiten Informationen, beispielsweise für eine Nutzung der zweiten Informationen durch die wenigstens eine Medikamentenausgabevorrichtung.

Bei weiteren beispielhaften Ausführungsformen ist vorgesehen, dass das Verfahren aufweist: Einlesen von dritten Informationen von wenigstens einem Teil eines Blisterbeutelschlauchs, wobei der wenigstens eine Teil des Blisterbeutelschlauchs beispielsweise wenigstens zwei, beispielsweise zueinander benachbarte, Blisterbeutel des Blisterbeutelschlauchs aufweist, Ermitteln der ersten Informationen basierend wenigstens auf den dritten Informationen.

Bei weiteren beispielhaften Ausführungsformen ist vorgesehen, dass für das Einlesen der dritten Informationen wenigstens eines der folgenden Elemente verwendet wird: a) eine, beispielsweise lokale, Leseeinrichtung der Medikamentenausgabevorrichtung, b) wenigstens eine weitere Leseeinrichtung, beispielsweise eine Leseeinrichtung eines Mobilgeräts, beispielsweise eines Smartphones oder Tablet-Computers, beispielsweise eines Benutzers, beispielsweise eines Pflegepersonals, c) ein manueller Einlesevorgang, beispielsweise durch einen Benutzer, beispielsweise ein Pflegepersonal.

Bei weiteren beispielhaften Ausführungsformen ist vorgesehen, dass für das Ermitteln der ersten Informationen wenigstens eines der folgenden Elemente verwendet wird: a) eine, beispielsweise lokale, Ermittlungseinrichtung der Medikamentenausgabevorrichtung, b) wenigstens eine weitere Ermittlungseinrichtung, beispielsweise eine entfernt zu der Medikamentenausgabevorrichtung angeordnete Ermittlungseinrichtung, beispielsweise eine Edge- und/oder Cloud-basierte Ermittlungseinrichtung, c) ein manueller Ermittlungsvorgang, beispielsweise durch einen Benutzer, beispielsweise ein Pflegepersonal, wobei beispielsweise das Ermitteln das Ausführen einer Zeichenerkennung, beispielsweise optischen Zeichenerkennung, beispielsweise OCR, aufweist.

Bei weiteren beispielhaften Ausführungsformen ist vorgesehen, dass das Verfahren aufweist: Fördern, beispielsweise Bewegen, wenigstens eines Teils eines bzw. des Blisterbeutelschlauchs, wobei der wenigstens eine Teil des Blisterbeutelschlauchs beispielsweise wenigstens zwei benachbarte Blisterbeutel des Blisterbeutelschlauchs aufweist, wobei beispielsweise das Fördern des wenigstens einen Teils eines bzw. des Blisterbeutelschlauchs wenigstens eines der folgenden Elemente aufweist: a) Fördern des wenigstens einen Teils des Blisterbeutelschlauchs mittels einer Fördereinrichtung der Medikamentenausgabevorrichtung, b) manuelles Fördern, beispielsweise durch einen Benutzer, beispielsweise ein Pflegepersonal.

Bei weiteren beispielhaften Ausführungsformen ist vorgesehen, dass das Bilden der zweiten Informationen aufweist: Bilden einer Liste, die Einnahmezeitpunkte des wenigstens einen Medikaments charakterisiert. Bei weiteren beispielhaften Ausführungsformen kann diese Liste z.B. durch die wenigstens eine Medikamentenausgabevorrichtung genutzt werden, beispielsweise um die Ausgabe von Medikamenten, z.B. gemäß einem Medikationsplan, zu steuern.

Bei weiteren beispielhaften Ausführungsformen ist vorgesehen, dass das Bereitstellen der zweiten Informationen wenigstens eines der folgenden Elemente aufweist: a) Speichern der zweiten Informationen, beispielsweise in Form einer Liste, in einer Speichereinrichtung der Medikamentenausgabevorrichtung, b) Senden der zweiten Informationen, beispielsweise über ein, z.B. zumindest bereichsweise öffentliches oder privates oder virtuelles privates, Netzwerk, beispielsweise an eine weitere Einheit, beispielsweise einen Edge-Server und/oder einen Cloud-Server bzw. ein z.B. wenigstens einen Cloud-Server und ein damit assoziiertes Netzwerk aufweisendes Cloud-System.

Bei weiteren beispielhaften Ausführungsformen ist vorgesehen, dass das Verfahren wenigstens eines der folgenden Elemente aufweist: a) Einlegen eines Blisterbeutelschlauchs in eine bzw. die Medikamentenausgabevorrichtung, b) Fördern wenigstens eines Teils des Blisterbeutelschlauchs, beispielsweise mittels einer bzw. der Fördereinrichtung der Medikamentenausgabevorrichtung, wobei der wenigstens eine Teil des Blisterbeutelschlauchs beispielsweise wenigstens zwei benachbarte Blisterbeutel des Blisterbeutelschlauchs aufweist, relativ zu einer Leseeinrichtung der Medikamentenausgabevorrichtung, c) Einlesen von dritten Informationen von wenigstens einem Teil des Blisterbeutelschlauchs, d) optional Ermitteln der zweiten Informationen basierend wenigstens auf den dritten Informationen, e) Senden der zweiten Informationen und/oder der dritten Informationen an wenigstens eine weitere Einheit, beispielsweise an einen Edge-Server und/oder einen Cloud-Server, beispielsweise über ein, z.B. zumindest bereichsweise öffentliches oder privates oder virtuelles privates, Netzwerk.

Bei weiteren beispielhaften Ausführungsformen kann somit z.B. eine vorhandene Medikamentenausgabevorrichtung dazu verwendet werden, die zweiten Informationen zu ermitteln und z.B. für die vorhandene Medikamentenausgabevorrichtung und/oder wenigstens eine weitere Medikamentenausgabevorrichtung und/oder z.B. ein Cloud-System bereitzustellen.

Bei weiteren beispielhaften Ausführungsformen ist vorgesehen, dass das Verfahren wenigstens eines der folgenden Elemente aufweist: a) Ermitteln einer ersten Identifikation, die mit einem ersten Blisterbeutel eines bzw. des Blisterbeutelschlauchs assoziiert ist, b) Ermitteln einer zweiten Identifikation, die mit einem weiteren, beispielsweise letzten, Blisterbeutel des Blisterbeutelschlauchs assoziiert ist, c) Ermitteln wenigstens einer weiteren Information des Blisterbeutelschlauchs, wobei beispielsweise die wenigstens eine weitere Information einen mit wenigstens einem Blisterbeutel des Blisterbeutelschlauchs assoziierten Einnahmezeitpunkt und/oder eine Identifikation des Blisterbeutelschlauchs charakterisiert, d) Bilden der zweiten Informationen, beispielsweise in Form einer Liste, basierend auf wenigstens einem der folgenden Elemente: d1) der ersten Identifikation, d2) der zweiten Identifikation, d3) der wenigstens einen weiteren Information.

Bei weiteren beispielhaften Ausführungsformen ist vorgesehen, dass das Verfahren wenigstens eines der folgenden Elemente aufweist: a) Erstellen wenigstens eines Digitalbilds und/oder Videos, wenigstens mancher, beispielsweise aller, Blisterbeutel des Blisterbeutelschlauchs, b) optional Senden des wenigstens einen Digitalbilds oder Videos an wenigstens eine weitere Einheit, beispielsweise an einen Edge-Server und/oder einen Cloud-Server, beispielsweise über ein, z.B. zumindest bereichsweise öffentliches oder privates oder virtuelles privates, Netzwerk, c) optional, Ermitteln der zweiten Informationen basierend auf dem wenigstens einen Digitalbild und/oder Video, d) Senden der zweiten Informationen an wenigstens eine weitere Einheit, beispielsweise an einen Edge-Server und/oder einen Cloud-Server, beispielsweise über ein, z.B. zumindest bereichsweise öffentliches oder privates oder virtuelles privates, Netzwerk.

Bei weiteren beispielhaften Ausführungsformen ist vorgesehen, dass für das Erstellen des wenigstens einen Digitalbilds und/oder Videos wenigstens eines der folgenden Elemente verwendet wird: a) Digitalkamera, b) Smartphone, beispielsweise in das Smartphone integrierte Kamera, c) Tablet, beispielsweise in das Tablet integrierte Kamera, d) Mobilgerät, beispielsweise in das Mobilgerät integrierte Kamera, e) Inspektionseinrichtung, beispielsweise einer Fertigungseinrichtung für den Blisterbeutelschlauch.

Weitere beispielhafte Ausführungsformen beziehen sich auf ein Verfahren, beispielsweise ein computerimplementiertes Verfahren, zum Verarbeiten von mit wenigstens einer Medikamentenausgabevorrichtung, beispielsweise zur Ausgabe von in Blisterbeuteln bzw. in einem Blisterbeutelschlauch angeordneten Medikamenten, assoziierten Daten, aufweisend: Empfangen von mit der wenigstens einen Medikamentenausgabevorrichtung assoziierten ersten Daten, und, optional, Senden wenigstens eines Teils der ersten Daten an die wenigstens eine Medikamentenausgabevorrichtung, wobei beispielsweise die ersten Daten wenigstens eines der folgenden Elemente aufweisen: a) erste Informationen, die zumindest Teile eines mit wenigstens einem Medikament assoziierten Medikationsplans charakterisieren, b) zweite Informationen für eine Steuerung der wenigstens einen Medikamentenausgabevorrichtung, c) dritte Informationen, die von wenigstens einem Teil eines Blisterbeutelschlauchs einlesbar sind.

Bei weiteren beispielhaften Ausführungsformen ist vorgesehen, dass das Verfahren aufweist: Bereitstellen einer, beispielsweise grafischen, Benutzeroberfläche zur Eingabe wenigstens eines Teils der ersten Daten, und, optional, Empfangen von mit der Benutzeroberfläche assoziierten Benutzereingaben.

Bei weiteren beispielhaften Ausführungsformen erfolgt das Bereitstellen der Benutzeroberfläche beispielsweise ein Bereitstellen von entsprechenden Benutzeroberflächenelementen, beispielsweise mittels des Hypertext Transfer Protocol (HTTP), beispielsweise über eine (virtuelle) private Netzwerkverbindung.

Bei weiteren beispielhaften Ausführungsformen ist vorgesehen, dass die Benutzeroberfläche wenigstens eines der folgenden Elemente aufweist: a) eine Übersicht über wenigstens einen, beispielsweise mittels eines Cloud-Systems verwaltbaren, Medikationsplan, b) eine Übersicht über, beispielsweise mittels eines Cloud-Systems verwaltbarer, Benutzer, beispielsweise Patienten, c) eine benutzerindividuelle, beispielsweise patientenindividuelle, Übersicht über wenigstens einen, beispielsweise mittels eines Cloud-Systems verwaltbaren, Medikationsplan, d) eine Eingabemaske zur Erstellung eines Medikationsplans für einen Benutzer, e) eine Eingabemaske zur Eingabe einer Identifikation eines Blisterbeutelschlauchs, f) eine Eingabemaske zur Eingabe von Einnahmezeitpunkten.

Weitere beispielhafte Ausführungsformen beziehen sich auf eine Vorrichtung zur Ausführung des Verfahrens gemäß den Ausführungsformen.

Weitere beispielhafte Ausführungsformen beziehen sich auf ein Mobilgerät, beispielsweise Smartphone, aufweisend eine Vorrichtung gemäß den Ausführungsformen.

Weitere beispielhafte Ausführungsformen beziehen sich auf eine Medikamentenausgabevorrichtung, beispielsweise zur Ausgabe von in Blisterbeuteln bzw. in einem Blisterbeutelschlauch angeordneten Medikamenten, aufweisend eine Vorrichtung gemäß den Ausführungsformen.

Weitere beispielhafte Ausführungsformen beziehen sich auf ein computerlesbares Speichermedium, umfassend Befehle, die bei der Ausführung durch einen Computer diesen veranlassen, das Verfahren gemäß den Ausführungsformen auszuführen.

Weitere beispielhafte Ausführungsformen beziehen sich auf ein Computerprogramm, umfassend Befehle, die bei der Ausführung des Programms durch einen Computer diesen veranlassen, das Verfahren gemäß den Ausführungsformen auszuführen.

Weitere beispielhafte Ausführungsformen beziehen sich auf ein Datenträgersignal, das das Computerprogramm gemäß den Ausführungsformen überträgt und/oder charakterisiert.

Weitere beispielhafte Ausführungsformen beziehen sich auf eine Verwendung des Verfahrens gemäß den Ausführungsformen und/oder der Vorrichtung gemäß den Ausführungsformen und/oder des Mobilgeräts gemäß den Ausführungsformen und/oder der Medikamentenausgabevorrichtung gemäß den Ausführungsformen und/oder des computerlesbaren Speichermediums gemäß den Ausführungsformen und/oder des Computerprogramms gemäß den Ausführungsformen und/oder des Datenträgersignals gemäß den Ausführungsformen für wenigstens eines der folgenden Elemente: a) Verarbeiten von mit wenigstens einer Medikamentenausgabevorrichtung assoziierten Daten, b) Bilden von Informationen für eine Steuerung wenigstens einer Medikamentenausgabevorrichtung, c) Übermitteln, beispielsweise Hochladen, von Informationen für eine Steuerung wenigstens einer Medikamentenausgabevorrichtung, beispielsweise zu einem Cloud-System, d) Bilden und/oder Speichern von Vorlagen für wenigstens einen Medikationsplan, e) Abgleichen von Daten, z.B. für einen Betrieb wenigstens einer Medikamentenausgabevorrichtung, f) Modifizieren von Daten, z.B. für einen Betrieb wenigstens einer Medikamentenausgabevorrichtung, g) Ermöglichen einer, beispielsweise automatischen, Überprüfung wenigstens eines Blisterbeutels, beispielsweise eines letzten Blisterbeutels, eines Blisterbeutelschlauchs, h) Nutzen von wenigstens einem Mobilgerät und/oder von wenigstens einer Medikamentenausgabevorrichtung und/oder von wenigstens einer weiteren Vorrichtung wie z.B. einer Inspektionseinrichtung einer Fertigungseinrichtung für Blisterbeutelschläuche zum Einlesen von Informationen von wenigstens einem Teil eines Blisterbeutelschlauchs, i) Zuordnen von Elementen eines Medikationsplans zu entsprechenden mit einer Produktion eines Blisterbeutelschlauchs assoziierten Elementen.

Weitere Merkmale, Anwendungsmöglichkeiten und Vorteile der Erfindung ergeben sich aus der nachfolgenden Beschreibung von Ausführungsbeispielen der Erfindung, die in den Figuren der Zeichnung dargestellt sind. Dabei bilden alle beschriebenen oder dargestellten Merkmale für sich oder in beliebiger Kombination den Gegenstand der Erfindung, unabhängig von ihrer Zusammenfassung in den Ansprüchen oder deren Rückbeziehung sowie unabhängig von ihrer Formulierung bzw. Darstellung in der Beschreibung bzw. in der Zeichnung.

In der Zeichnung zeigt:
- Fig. 1: schematisch ein Flussdiagramm gemäß beispielhaften Ausführungsformen,
- Fig. 2: schematisch ein Flussdiagramm gemäß beispielhaften Ausführungsformen,
- Fig. 3: schematisch ein Blockdiagramm gemäß beispielhaften Ausführungsformen,
- Fig. 4: schematisch ein Blockdiagramm gemäß beispielhaften Ausführungsformen,
- Fig. 5: schematisch ein Flussdiagramm gemäß beispielhaften Ausführungsformen,
- Fig. 6: schematisch ein Flussdiagramm gemäß beispielhaften Ausführungsformen,
- Fig. 7: schematisch ein Flussdiagramm gemäß beispielhaften Ausführungsformen,
- Fig. 8: schematisch Blisterbeutel gemäß beispielhaften Ausführungsformen,
- Fig. 9: schematisch ein Flussdiagramm gemäß beispielhaften Ausführungsformen,
- Fig. 10: schematisch ein Flussdiagramm gemäß beispielhaften Ausführungsformen,
- Fig. 11: schematisch ein Flussdiagramm gemäß beispielhaften Ausführungsformen,
- Fig. 12: schematisch ein Flussdiagramm gemäß beispielhaften Ausführungsformen,
- Fig. 13: schematisch ein Flussdiagramm gemäß beispielhaften Ausführungsformen,
- Fig. 14: schematisch ein Blockdiagramm gemäß beispielhaften Ausführungsformen,
- Fig. 15: schematisch ein Blockdiagramm gemäß beispielhaften Ausführungsformen,
- Fig. 16: schematisch ein Blockdiagramm gemäß beispielhaften Ausführungsformen,
- Fig. 17: schematisch Aspekte von Verwendungen gemäß beispielhaften Ausführungsformen.

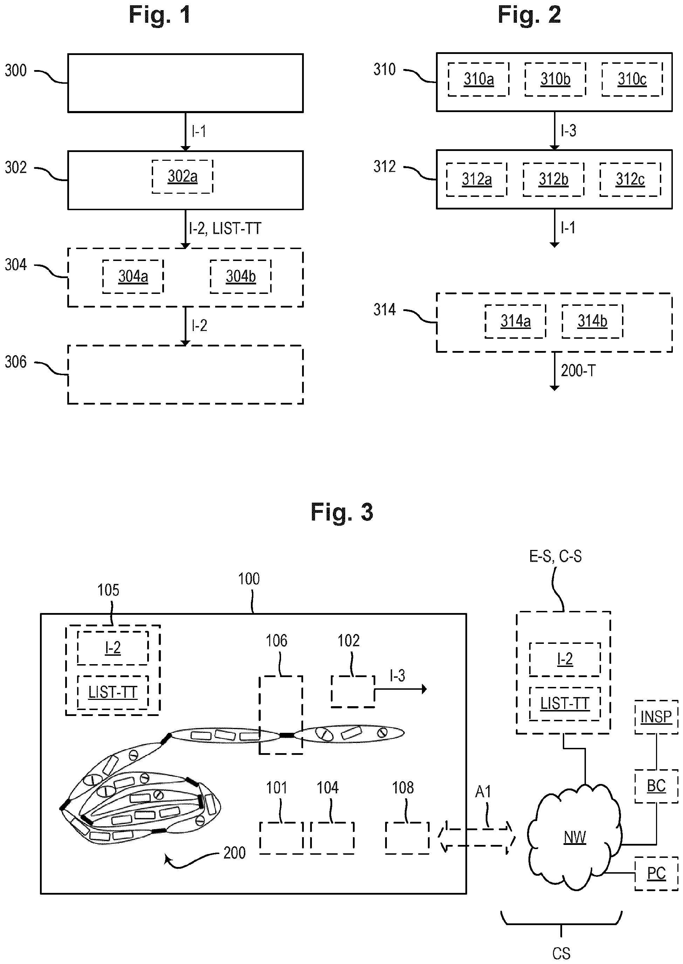

Beispielhafte Ausführungsformen, Fig. 1, beziehen sich auf ein Verfahren, beispielsweise ein computerimplementiertes Verfahren, zum Verarbeiten von mit wenigstens einer Medikamentenausgabevorrichtung 100 (Fig. 3), beispielsweise zur Ausgabe von in Blisterbeuteln bzw. in einem Blisterbeutelschlauch 200 angeordneten Medikamenten, assoziierten Daten, aufweisend: Ermitteln 300 (Fig. 1) von ersten Informationen I-1, die zumindest Teile eines mit wenigstens einem Medikament assoziierten Medikationsplans charakterisieren, Bilden 302 von zweiten Informationen I-2 für eine Steuerung der wenigstens einen Medikamentenausgabevorrichtung 100 (Fig. 3) basierend wenigstens auf den ersten Informationen I-1, und, optional, Bereitstellen 304 (Fig. 1) der zweiten Informationen I-2, beispielsweise für eine, z.B. optionale, Nutzung 306 der zweiten Informationen I-2 durch die wenigstens eine Medikamentenausgabevorrichtung 100.

Bei weiteren beispielhaften Ausführungsformen ist vorgesehen, dass die Medikamentenausgabevorrichtung 100 dazu ausgebildet ist, in Blisterbeuteln angeordnete Medikamente auszugeben. Ein Blisterbeutelschlauch 200 (Fig. 3) oder kurz "Blisterschlauch" ist ein im Wesentlichen bandförmiges Erzeugnis, bei welchem eine Mehrzahl von einzelnen Verpackungseinheiten, die auch als Blisterbeutel bzw. Schlauchblister bezeichnet werden, hintereinander angeordnet sind. Ein Blisterschlauch kann beispielsweise als Medikamentenblisterschlauch ausgeführt sein, der in den einzelnen Blisterbeuteln jeweils ein oder mehrere Medikamente wie z.B. Tabletten aufweist. Beispielsweise kann die Medikamentenausgabevorrichtung 100 ein oder mehrere Blisterbeutelschläuche 200 ausgeben.

Bei weiteren beispielhaften Ausführungsformen, Fig. 3, kann die Medikamentenausgabevorrichtung 100 beispielsweise eine Steuerungseinrichtung 101 aufweisen, die z.B. zumindest zeitweise einen Betrieb wenigstens einer Komponente der Medikamentenausgabevorrichtung 100 steuert. Beispielsweise kann die Medikamentenausgabevorrichtung 100 dazu ausgebildet sein, Medikamente, z.B. enthalten in einzelnen Blisterbeuteln des Blisterbeutelschlauchs 200, basierend auf den vorstehend bereits erwähnten zweiten Informationen I-2 auszugeben.

Bei weiteren beispielhaften Ausführungsformen weist die Medikamentenausgabevorrichtung 100 wenigstens eines der folgenden Elemente auf: a) eine Leseeinrichtung 102 zum Einlesen von dritten Informationen I-3, die z.B. auf dem Blisterbeutelschlauch 200 angeordnet (z.B. aufgedruckt) sind, b) eine Ermittlungseinrichtung 104 für das Ermitteln 300 (Fig. 1) der ersten Informationen I-1, c) eine Speichereinrichtung 105, beispielsweise für die zweiten Informationen I-2, d) eine Fördereinrichtung 106 zum Fördern wenigstens eines Teils des Blisterbeutelschlauchs, beispielsweise für eine Ausgabe wenigstens eines Blisterbeutels, e) eine Datenschnittstelle 108 zum Datenaustausch A1 mit wenigstens einer externen Einheit, z.B. mit einem Cloud-System CS, wobei das Cloud-System CS beispielsweise wenigstens einen Edge-Server E-S und/oder einen Cloud-Server C-S aufweist und ein Netzwerk NW.

Bei weiteren beispielhaften Ausführungsformen kann eine Fertigungseinrichtung BC für Blisterbeutelschläuche 200, die z.B. auch als "Blistercenter" bezeichnet werden kann, an das Cloud-System CS angebunden sein, so dass zumindest bei manchen Ausführungsformen zumindest zeitweise Daten von dem Blistercenter BC zu dem Cloud-System CS übertragbar sind, beispielsweise mit einer Fertigung der Blisterbeutelschläuche 200 assoziierte Daten (z.B. Identifikation einzelner Blisterbeutel und/oder eines jeweiligen Blisterbeutelschlauchs, die z.B. im Rahmen der Fertigung zugeordnet werden).

Bei weiteren beispielhaften Ausführungsformen weisen die mit der Fertigung der Blisterbeutelschläuche 200 assoziierten Daten z.B. mit einem oder mehreren Medikationsplänen assoziierte Daten ("Medikationsplan-Daten") auf, die von dem Blistercenter BC z.B. an das Cloud-System CS übertragbar sind. Beispielsweise lädt das Blistercenter BC die Daten, z.B. über eine definierte Schnittstelle, auf wenigstens einen Server E-S, C-S des Cloud-Systems CS, damit diese Daten z.B. für einen Betrieb wenigstens einer Medikamentenausgabevorrichtung 100, z.B. bei einem Benutzer, z.B. Patienten, zur Verfügung stehen.

Bei weiteren beispielhaften Ausführungsformen kann, z.B. für eine Absicherung einer Zuordnung Gerät - Patient - Medikation, z.B. beim Einlegen des eines Blisterbeutelschlauchs 200 in die Medikamentenausgabevorrichtung 100, beispielsweise durch ein Pflegepersonal, eine Identität des Patienten mit einem z.B. eindeutigen Code auf den Blisterbeuteln und / oder mit durch Menschen lesbaren Informationen, z.B. ein Text mit dem Namen des Patienten und/oder mit Einnahmezeitpunkten der Medikamente, und z.B. einer in dem Cloud-System CS hinterlegten Seriennummer der Medikamentenausgabevorrichtung 100 abgeglichen werden.

Bei weiteren beispielhaften Ausführungsformen kann nach einem erfolgreichen Abgleich beispielsweise ein Medikationsplan bzw. die zweiten Informationen I-2, die bei manchen beispielhaften Ausführungsformen auch als "Beutelausgabe-Informationen" bezeichenbar sind, aus der Cloud, z.B. von dem Cloud-System CS, auf die Medikamentenausgabevorrichtung 100 übertragen werden, z.B. über das Netzwerk NW und die Datenschnittstelle 108.

Bei weiteren beispielhaften Ausführungsformen können basierend auf der Übertragung der Daten (z.B. aufweisend Medikationsdaten) von dem Blistercenter BC zu dem Cloud-System CS die zweiten Informationen I-2, z.B. in Form einer Liste LIST-TT, die z.B. u.a. Einnahmezeitpunkte von mit den einzelnen Blisterbeuteln eines Blisterbeutelschlauchs 200 charakterisiert, in der Cloud, z.B. in dem Cloud-System CS, hinterlegt, z.B. zumindest zeitweise gespeichert, und z.B. über das Netzwerk NW auch an die Medikamentenausgabevorrichtung 100 übertragen werden, wobei die Medikamentenausgabevorrichtung 100 die auf diese Weise erhaltenen Informationen I-2 bzw. Liste LIST-TT z.B. zur Ausgabe von Blisterbeuteln z.B. eines zuvor eingelegten Blisterbeutelschlauchs 200 verwenden kann.

Bei weiteren beispielhaften Ausführungsformen werden die von dem Cloud-System CS erhaltenen zweiten Informationen I-2 bzw. die Liste LIST-TT, z.B. für einen aktuell in die Medikamentenausgabevorrichtung 100 eingelegten Blisterbeutelschlauch 200, zumindest zeitweise in der Medikamentenausgabevorrichtung 100, beispielsweise mittels der Speichereinrichtung 105, gespeichert, so dass ein eventueller Stromausfall oder eine ausfallende bzw. fehlende Datenverbindung, beispielsweise Internetverbindung, A1 keine Auswirkungen auf den Betrieb der Medikamentenausgabevorrichtung 100, z.B. auf die darin hinterlegten zweiten Informationen I-2, z.B. aufweisend AusgabeZeiten und auf die Medikamenten-Ausgabe haben.

Bei weiteren beispielhaften Ausführungsformen kann, z.B. nach jeder Ausgabe eines Blisterbeutels, die Liste LIST-TT, beispielsweise in der Medikamentenausgabevorrichtung 100, aktualisiert werden. Optional kann z.B. auch die aktualisierte Liste in das Cloud-System CS übertragen werden, beispielsweise zur Dokumentation eines Betriebs der Medikamentenausgabevorrichtung 100.

Bei weiteren beispielhaften Ausführungsformen weist das Netzwerk NW wenigstens eine drahtgebundene Datenübertragung (z.B. Ethernet, Internet) und/oder wenigstens eine drahtlose Datenübertragung (z.B. WLAN, zelluläres Mobilfunksystem z.B. vom 4G- oder 5G-Typ) auf.

Bei weiteren beispielhaften Ausführungsformen können, z.B. dann, wenn Datenübertragungen, z.B. ein Datenupload, von dem Blistercenter BC zu dem Cloud-System CS, nicht möglich sind, z.B. aufgrund einer nicht vorhandenen Anbindung an das Cloud-System CS und/oder zumindest vorübergehender Störungen der Datenübertragung, ein oder mehrere der nachfolgend beispielhaft beschriebenen Aspekte und/oder Ausführungsformen vorgesehen werden, die z.B. einzeln für sich oder in Kombination miteinander mit wenigstens einem der vorstehend beispielhaft beschriebenen Aspekte kombinierbar sind.

Bei weiteren beispielhaften Ausführungsformen, Fig. 2, ist vorgesehen, dass das Verfahren aufweist: Einlesen 310 von dritten Informationen I-3 (s. auch Fig. 3) von wenigstens einem Teil eines Blisterbeutelschlauchs 200, wobei der wenigstens eine Teil des Blisterbeutelschlauchs 200 beispielsweise wenigstens zwei, beispielsweise zueinander benachbarte, Blisterbeutel des Blisterbeutelschlauchs 200 aufweist, Ermitteln 312 (Fig. 2) der ersten Informationen I-1 basierend wenigstens auf den dritten Informationen I-3.

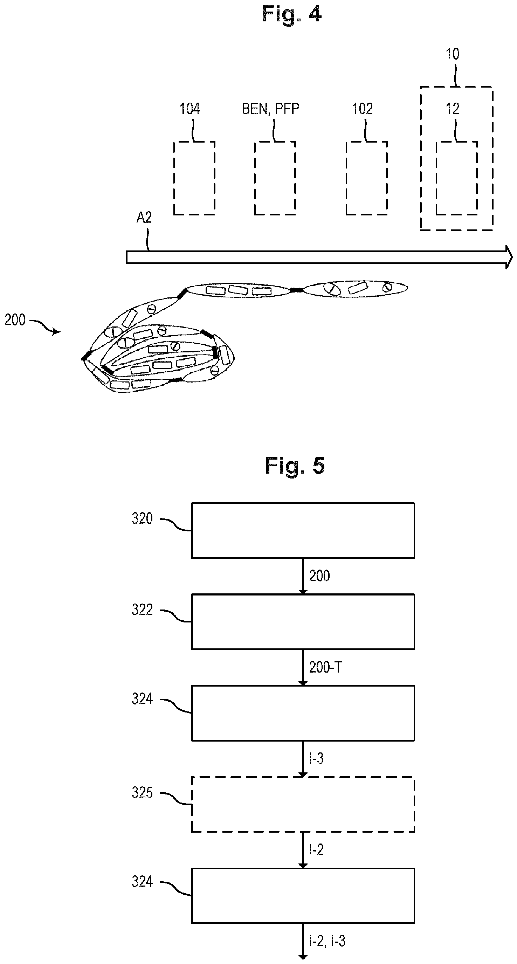

Bei weiteren beispielhaften Ausführungsformen, Fig. 4, ist vorgesehen, dass für das Einlesen 310 (Fig. 2) der dritten Informationen I-3 wenigstens eines der folgenden Elemente verwendet wird: a) eine, beispielsweise lokale, Leseeinrichtung 102 der Medikamentenausgabevorrichtung 100, s. auch Block 310a gemäß Fig. 2, b) wenigstens eine weitere Leseeinrichtung 12, beispielsweise eine Leseeinrichtung 12 eines Mobilgeräts 10, beispielsweise eines Smartphones oder Tablet-Computers, beispielsweise eines Benutzers BEN, beispielsweise eines Pflegepersonals PFP, s. auch Block 310b gemäß Fig. 2, c) ein manueller Einlesevorgang, beispielsweise durch einen Benutzer BEN, beispielsweise ein Pflegepersonal PFP, s. auch Block 310c gemäß Fig. 2.

Bei weiteren beispielhaften Ausführungsformen, Fig. 2, ist vorgesehen, dass für das Ermitteln 312 der ersten Informationen I-1 wenigstens eines der folgenden Elemente verwendet wird: a) eine, beispielsweise lokale, Ermittlungseinrichtung 312a der Medikamentenausgabevorrichtung 100, s. z.B. Element 104 gemäß Fig. 3, b) wenigstens eine weitere Ermittlungseinrichtung 312b (Fig. 2), beispielsweise eine entfernt zu der Medikamentenausgabevorrichtung 100 angeordnete Ermittlungseinrichtung, beispielsweise eine Edge- und/oder Cloud-basierte Ermittlungseinrichtung des Cloud-Systems CS, c) ein manueller Ermittlungsvorgang 312c (Fig. 2), beispielsweise durch einen Benutzer BEN, beispielsweise ein Pflegepersonal, PFP, wobei beispielsweise das Ermitteln 312 das Ausführen einer Zeichenerkennung, beispielsweise optischen Zeichenerkennung, beispielsweise OCR, aufweist, z.B. dann, wenn einer der Aspekte 312a, 312b verwendet wird.

Bei weiteren beispielhaften Ausführungsformen, Fig. 2, ist vorgesehen, dass das Verfahren aufweist: Fördern 314, beispielsweise Bewegen, wenigstens eines Teils 200-T eines bzw. des Blisterbeutelschlauchs 200, s. auch den Pfeil A2 gemäß Fig. 4, wobei der wenigstens eine Teil des Blisterbeutelschlauchs 200 beispielsweise wenigstens zwei benachbarte Blisterbeutel des Blisterbeutelschlauchs 200 aufweist, wobei beispielsweise das Fördern 314 des wenigstens einen Teils 200-T eines bzw. des Blisterbeutelschlauchs 200 wenigstens eines der folgenden Elemente aufweist: a) Fördern 314a des wenigstens einen Teils 200-T des Blisterbeutelschlauchs 200 mittels einer Fördereinrichtung 106 (Fig. 3) der Medikamentenausgabevorrichtung 100, b) manuelles Fördern 314b, beispielsweise durch einen Benutzer BEN (Fig. 4), beispielsweise ein Pflegepersonal PFP.

Bei weiteren beispielhaften Ausführungsformen, Fig. 4, können durch das Fördern 314 (Fig. 2) mehrere, beispielsweise alle, Blisterbeutel des Blisterbeutelschlauchs 200 z.B. an wenigstens einer Leseeinrichtung 102, 12 vorbeibewegt werden, was z.B. ein effizientes Einlesen der dritten Informationen I-3 ermöglicht. Auf dieser Basis können dann beispielsweise die ersten Informationen I-1 ermittelt werden, s. z.B. Block 312 gemäß Fig. 2.

Bei weiteren beispielhaften Ausführungsformen, Fig. 1, ist vorgesehen, dass das Bilden 302 der zweiten Informationen I-2 aufweist: Bilden 302a einer Liste LIST-TT, die z.B. Einnahmezeitpunkte des wenigstens einen Medikaments charakterisiert. Bei weiteren beispielhaften Ausführungsformen kann diese Liste LIST-TT z.B. durch die wenigstens eine Medikamentenausgabevorrichtung 100 genutzt werden, beispielsweise um die Ausgabe von Medikamenten, z.B. gemäß einem Medikationsplan, zu steuern.

Bei weiteren beispielhaften Ausführungsformen, Fig. 1, ist vorgesehen, dass das Bereitstellen 304 der zweiten Informationen I-2 wenigstens eines der folgenden Elemente aufweist: a) Speichern 304a der zweiten Informationen I-2, beispielsweise in Form einer Liste LIST-TT, in einer Speichereinrichtung 105 (Fig. 3) der Medikamentenausgabevorrichtung 100, b) Senden 304b (Fig. 1) der zweiten Informationen I-2, beispielsweise über ein, z.B. zumindest bereichsweise öffentliches oder privates oder virtuelles privates, Netzwerk NW (Fig. 3), beispielsweise an eine weitere Einheit, beispielsweise einen Edge-Server E-S und/oder einen Cloud-Server C-S bzw. ein z.B. wenigstens einen Cloud-Server C-S und ein damit assoziiertes Netzwerk NW aufweisendes Cloud-System CS.

Bei weiteren beispielhaften Ausführungsformen, Fig. 5, ist vorgesehen, dass das Verfahren wenigstens eines der folgenden Elemente aufweist: a) Einlegen 320 eines Blisterbeutelschlauchs 200 in eine bzw. die Medikamentenausgabevorrichtung 100 (Fig. 2), b) Fördern 322 wenigstens eines Teils 200-T des Blisterbeutelschlauchs, beispielsweise mittels einer bzw. der Fördereinrichtung 106 der Medikamentenausgabevorrichtung 100, wobei der wenigstens eine Teil 200-T des Blisterbeutelschlauchs 200 beispielsweise wenigstens zwei benachbarte Blisterbeutel des Blisterbeutelschlauchs aufweist, relativ zu einer Leseeinrichtung 102 der Medikamentenausgabevorrichtung 100, c) Einlesen 324 von dritten Informationen I-3 von wenigstens einem Teil des Blisterbeutelschlauchs 200, d) optional Ermitteln 325 der zweiten Informationen I-2 basierend wenigstens auf den dritten Informationen I-3, e) Senden 324 der zweiten Informationen I-2 und/oder der dritten Informationen I-3 an wenigstens eine weitere Einheit, beispielsweise an einen Edge-Server E-S und/oder einen Cloud-Server C-S, beispielsweise über das Netzwerk NW (Fig. 3).

Bei weiteren beispielhaften Ausführungsformen kann somit z.B. eine vorhandene Medikamentenausgabevorrichtung 100 (Fig. 3) dazu verwendet werden, die zweiten Informationen I-2 effizient zu ermitteln und z.B. für die vorhandene Medikamentenausgabevorrichtung 100 selbst und/oder wenigstens eine weitere Medikamentenausgabevorrichtung und/oder z.B. ein Cloud-System CS bereitzustellen.

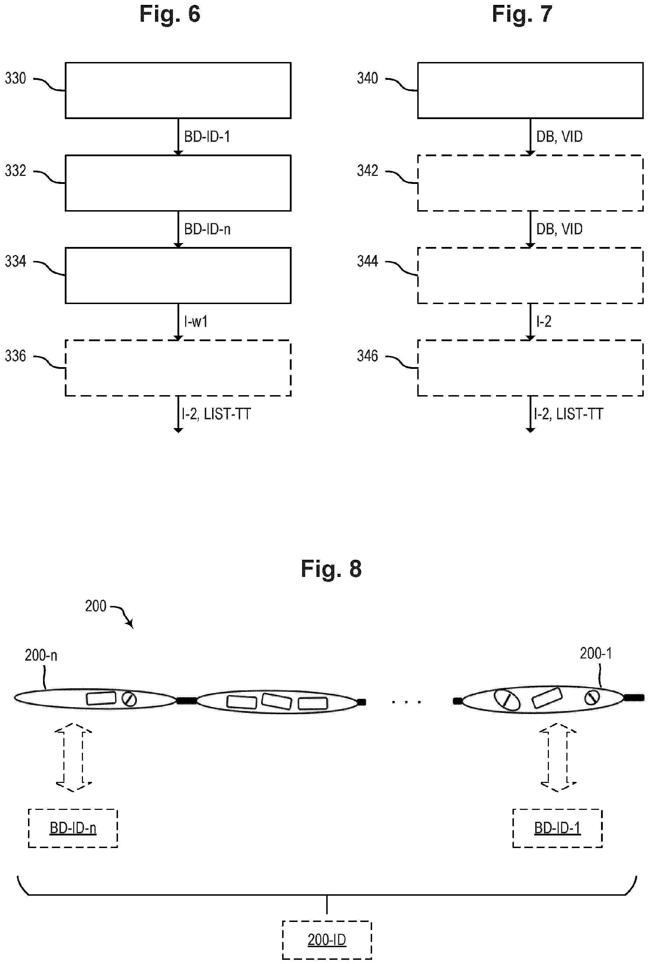

Bei weiteren beispielhaften Ausführungsformen, Fig. 6, ist vorgesehen, dass das Verfahren wenigstens eines der folgenden Elemente aufweist: a) Ermitteln 330 einer ersten Identifikation BD-ID-1, die mit einem ersten Blisterbeutel 200-1 (Fig. 8) eines bzw. des Blisterbeutelschlauchs 200 assoziiert ist, b) Ermitteln 332 (Fig. 6) einer zweiten Identifikation BD-ID-n, die mit einem weiteren, beispielsweise letzten, Blisterbeutel 200-n des Blisterbeutelschlauchs 200 assoziiert ist, c) Ermitteln 334 wenigstens einer weiteren Information I-w1 des Blisterbeutelschlauchs 200, wobei beispielsweise die wenigstens eine weitere Information I-w1 einen mit wenigstens einem Blisterbeutel 200-1, 200-n des Blisterbeutelschlauchs 200 assoziierten Einnahmezeitpunkt und/oder eine Identifikation 200-ID des Blisterbeutelschlauchs 200 charakterisiert, d) Bilden 336 der zweiten Informationen I-2, beispielsweise in Form einer bzw. der Liste LIST-TT, basierend auf wenigstens einem der folgenden Elemente: d1) der ersten Identifikation BD-ID-1, d2) der zweiten Identifikation BD-ID-n, d3) der wenigstens einen weiteren Information I-w1.

Bei weiteren beispielhaften Ausführungsformen ist die Identifikation BD-ID-1 z.B. ein durch Menschen und/oder Maschinen lesbarer Code, z.B. optischer Code, z.B. ein- oder mehrdimensionaler Barcode, z.B. QR-Code, und/oder ein magnetischer Code, und/oder ein sonstiger elektrisch und/oder magnetisch und/oder elektromagnetisch und/oder optisch lesbarer Code. Beispielsweise kann die Identifikation z.B. auch mittels bzw. in Form eines NFC (near field communication) tags an einem Blisterbeutel angeordnet sein.

Bei weiteren beispielhaften Ausführungsformen können wenigstens manche z.B. im Rahmen des beispielhaften Ablaufs gemäß Fig. 6 ermittelte Information BD-ID-1, BD-ID-n, I-w1, I-2, LIST-TT bzw. damit assoziierte Daten z.B. an das Cloud-System CS gesendet bzw. zumindest zeitweise in dem Cloud-System CS gespeichert werden, z.B. für einen Abruf durch die wenigstens eine Medikamentenausgabevorrichtung 100 bzw. für ein Senden an die wenigstens eine Medikamentenausgabevorrichtung 100.

Bei weiteren beispielhaften Ausführungsformen, Fig. 7, ist vorgesehen, dass das Verfahren wenigstens eines der folgenden Elemente aufweist: a) Erstellen 340 wenigstens eines Digitalbilds DB und/oder (z.B. digitalen) Videos VID, wenigstens mancher, beispielsweise aller, Blisterbeutel 200-1, ..., 200-n des Blisterbeutelschlauchs 200 (Fig. 8), b) optional Senden 342 (Fig. 7) des wenigstens einen Digitalbilds DB und/oder Videos VID an wenigstens eine weitere Einheit, beispielsweise an einen Edge-Server E-S (Fig. 3) und/oder einen Cloud-Server C-S, beispielsweise über ein, z.B. zumindest bereichsweise öffentliches oder privates oder virtuelles privates, Netzwerk NW, c) optional, Ermitteln 344 der zweiten Informationen I-2 basierend auf dem wenigstens einen Digitalbild DB und/oder Video VID, d) Senden 346 der zweiten Informationen I-2 an wenigstens eine weitere Einheit, beispielsweise an einen Edge-Server E-S und/oder einen Cloud-Server C-S, beispielsweise über das Netzwerk NW.

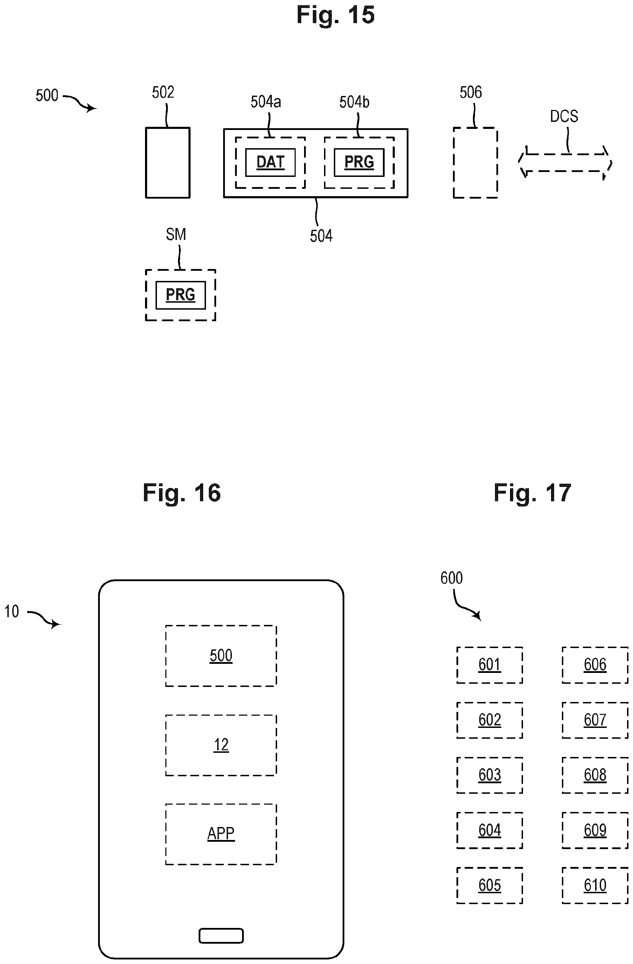

Bei weiteren beispielhaften Ausführungsformen, Fig. 7, ist vorgesehen, dass für das Erstellen 340 des wenigstens einen Digitalbilds DB und/oder Videos VID wenigstens eines der folgenden Elemente verwendet wird: a) Digitalkamera, b) Smartphone 10 (Fig. 16), beispielsweise in das Smartphone 10 integrierte Kamera 12, c) Tablet, beispielsweise in das Tablet integrierte Kamera, d) (sonstiges) Mobilgerät, beispielsweise in das Mobilgerät integrierte Kamera, e) Inspektionseinrichtung INSP (Fig. 2), beispielsweise einer Fertigungseinrichtung BC für den Blisterbeutelschlauch 200.

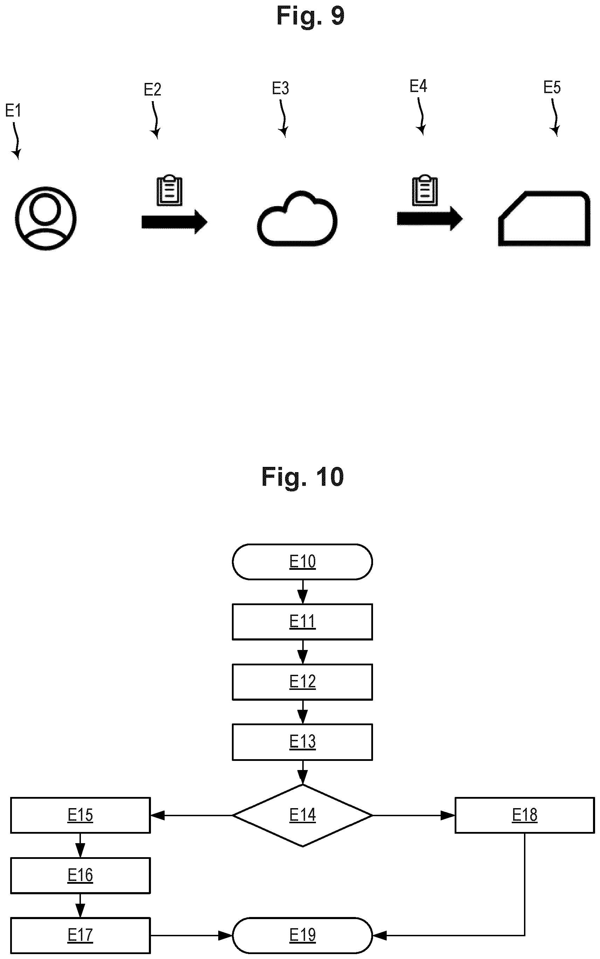

Fig. 9 zeigt schematisch ein Flussdiagramm, das einen Datenfluss gemäß weiteren beispielhaften Ausführungsformen beschreibt. Element E1 symbolisiert ein Pflegepersonal, Element E2 symbolisiert Daten, z.B. einen Medikationsplan charakterisierende Daten, die von dem Pflegepersonal E1 an ein Cloud-System E3, z.B. zumindest ähnlich zu dem Cloud-System CS gemäß Fig. 3, übertragen werden. Element E4 symbolisiert eine Übertragung von Daten, z.B. von den Medikationsplan charakterisierenden Daten, von dem Cloud-System E3 an wenigstens eine Medikamentenausgabevorrichtung E5, z.B. zumindest ähnlich zu der Medikamentenausgabevorrichtung 100 gemäß Fig. 3. Mit dem beispielhaft gemäß Fig. 9 beschriebenen Ablauf können z.B. Medikationspläne effizient für einzelne Benutzer, beispielsweise Patienten, z.B. auf jeweiligen Medikamentenausgabevorrichtungen E5, 100 bereitgestellt werden.

Fig. 10 zeigt schematisch ein Flussdiagramm gemäß weiteren beispielhaften Ausführungsformen. Der gezeigte Ablauf ermöglicht beispielsweise ein Hochladen von mit wenigstens einem Blisterbeutelschlauch 200 (Fig. 8) assoziierten Daten, beispielsweise in das Cloud-System CS, E3.

Element E10 symbolisiert einen Start einer Eingabe von z.B. einen Blisterbeutelschlauch 200 charakterisierenden Daten, beispielsweise durch ein Pflegepersonal, beispielsweise unter Verwendung eines Computers, z.B. Personal Computers (PC), beispielsweise über eine Datenschnittstelle, z.B. Internet- bzw. Web-Interface, z.B. des Cloud-Systems CS, E3.

Element E11 symbolisiert ein Eingeben von einem oder mehreren Einnahmezeitpunkten für mit dem Blisterbeutelschlauch 200 assoziierte Medikamente, beispielsweise basierend auf einem Medikationsplan.

Element E12 symbolisiert ein Eingeben eines Startdatums und/oder einer Startzeit, die z.B. mit einem ersten Blisterbeutel 200-1 (Fig. 8) des Blisterbeutelschlauchs 200 assoziiert sind. Beispielsweise können bei weiteren beispielhaften Ausführungsformen das Startdatum und/oder die Startzeit manuell, z.B. durch das Pflegepersonal, erfasst, z.B. von dem ersten Blisterbeutel 200-1 abgelesen, und dann gemäß Element E12 eingegeben werden. Ein Ablesen unter Verwendung eines technischen Geräts, z.B. Mobilgeräts, z.B. Smartphones 10 (Fig. 16), mit einer Lesevorrichtung 12, ist bei weiteren beispielhaften Ausführungsformen ebenfalls denkbar. Bei weiteren beispielhaften Ausführungsformen kann damit z.B. auch ein (nur) maschinenlesbarer Code decodiert und dann z.B. durch das Pflegepersonal in Form von Text, z.B. Klartext, eingegeben werden.

Element E13 symbolisiert, beispielsweise ähnlich zu Element E12, ein Eingeben eines Enddatums und/oder einer Endzeit, die z.B. mit einem letzten Blisterbeutel 200-n (Fig. 8) des Blisterbeutelschlauchs 200 assoziiert sind. Beispielsweise können bei weiteren beispielhaften Ausführungsformen das Enddatum und/oder die Endzeit manuell, z.B. durch das Pflegepersonal, erfasst, z.B. von dem letzten Blisterbeutel 200-n abgelesen, und dann gemäß Element E13 eingegeben werden. Ein Ablesen unter Verwendung eines technischen Geräts, z.B. Mobilgeräts, z.B. Smartphones 10 (Fig. 16), mit einer Lesevorrichtung 12, ist bei weiteren beispielhaften Ausführungsformen ebenfalls denkbar. Bei weiteren beispielhaften Ausführungsformen kann damit z.B. auch ein (nur) maschinenlesbarer Code decodiert und dann z.B. durch das Pflegepersonal in Form von Text, z.B. Klartext, eingegeben werden.

Element E14 symbolisiert das Ermitteln, ob einzelne Blisterbeutel des Blisterbeutelschlauchs 200 identifizierende Daten (z.B. Blisterbeutel-IDs, z.B. "pouch IDs") eingegeben werden sollen. Falls ja, wird zu Element E15 verzweigt, das das Eingeben von den ersten Blisterbeutel 200-1 identifizierenden Daten BD-ID-1 zum Gegenstand hat. Element E16 symbolisiert das Eingeben von den letzten Blisterbeutel 200-n identifizierenden Daten BD-ID-n. Es ist zu beachten, dass der beispielhafte Ablauf gemäß den Elementen E14, E15, beispielsweise nur, das Eingeben der den ersten Blisterbeutel 200-1 identifizierenden Daten BD-ID-1 und der den letzten Blisterbeutel 200-n identifizierenden Daten BD-ID-n vorsieht, nicht jedoch zum Beispiel das Eingeben von weiteren Daten, die z.B. wenigstens einen in dem Blisterbeutelschlauch 200 zwischen dem ersten Blisterbeutel 200-1 und dem letzten Blisterbeutel 200-n liegenden Blisterbeutel charakterisieren.

Element E17 symbolisiert das Ermitteln, z.B. Bilden, eine Sequenz von Identifikationen für mehrere, beispielsweise alle, Blisterbeutel des betrachteten Blisterbeutelschlauchs 200 basierend wenigstens auf den zuvor eingegebenen Daten BD-ID-1, BD-ID-2 (und ggf. basierend auf optionalem a-priori-Wissen, z.B. über eine Anzahl von Blisterbeuteln in dem Blisterbeutelschlauch 200, usw.).

Element E19 symbolisiert ein Hochladen wenigstens eines Teils der Daten, die beispielhaft unter Bezugnahme auf Fig. 10 beschriebenen worden sind, beispielsweise in das Cloud-System CS, E3.

Sofern das Ermitteln E14 ergibt, dass keine Daten, die einzelne Blisterbeutel des Blisterbeutelschlauchs 200 identifizieren, eingegeben werden sollen, wird von Element E14 direkt zu Element E18 verzweigt, das eine Ermittlung, beispielsweise Bildung, eines Medikationsplans zum Gegenstand hat, der in Block E19 beispielsweise in das Cloud-System CS, E3 hochgeladen wird.

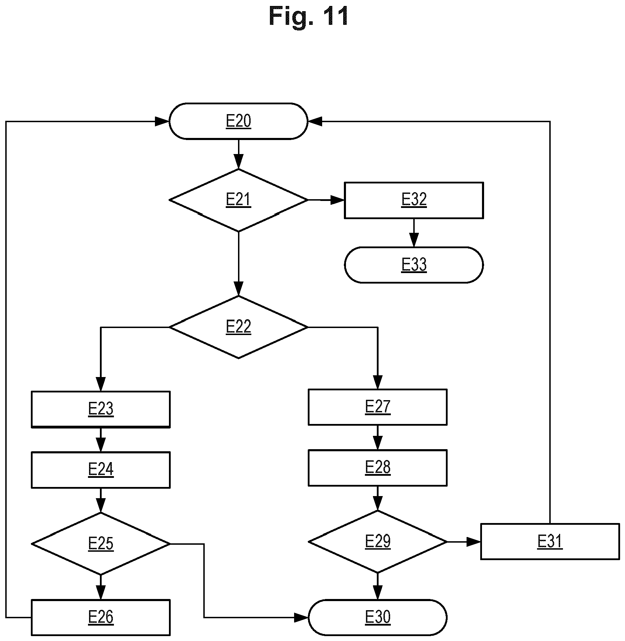

Fig. 11 zeigt schematisch ein Flussdiagramm gemäß weiteren beispielhaften Ausführungsformen. Der gezeigte Ablauf ist beispielsweise bei einem Einlegen eines (z.B. neuen) Blisterbeutelschlauchs 200 (Fig. 8), z.B. in zumindest teilweise aufgerollter Form, z.B. als Blisterbeutelrolle (s. Fig. 3), in die Medikamentenausgabevorrichtung 100 verwendbar.

Element E20 symbolisiert einen Beginn des Ablaufs, der der Medikamentenausgabevorrichtung 100 beispielsweise durch Auswahl einer entsprechenden Funktion, beispielsweise über eine, beispielsweise grafische, Benutzerschnittstelle signalisierbar ist, beispielsweise durch Betätigen eines entsprechenden Benuzterschnittstellenelements, z.B. eines grafischen Benuzterschnittstellenelements auf einer berührungsempfindlichen Anzeigevorrichtung (nicht gezeigt).

Element E21 symbolisiert ein Ermitteln, ob beispielsweise Daten (z.B. in Form der zweiten Informationen bzw. der Liste LIST-TT) für einen Betrieb der Medikamentenausgabevorrichtung 100 mit dem eingelegten z.B. neuen Blisterbeutelschlauch, beispielsweise gemäß weiteren beispielhaften Ausführungsformen, vorhanden sind, beispielsweise durch ein vorangehendes Hochladen bzw. Bereitstellen, z.B. über das Cloud-System CS, E3.

Falls ja, wird zu Element E22 verzweigt, das ein Ermitteln symbolisiert, ob die hochgeladenen Daten (z.B. in Form der zweiten Informationen bzw. der Liste LIST-TT) einzelne Blisterbeutel des Blisterbeutelschlauchs 200 identifizierende Daten (z.B. Blisterbeutel-IDs, z.B. "pouch IDs") aufweisen. Falls ja, wird zu Element E23 verzweigt, das das Laden eines Medikationsplans bzw. der Liste LIST-TT, z.B. aus der Cloud, z.B. von dem Cloud-System CS, symbolisiert.

Element E24 symbolisiert ein Einlesen von Informationen I-3 des eingelegten Blisterbeutelschlauchs 200, beispielsweise das Einlesen eines eine Identifikation des ersten Blisterbeutel 200-1 charakterisierenden Barcodes, z.B. mittels der Leseeinrichtung 102 (Fig. 3).

Element E25 symbolisiert ein Ermitteln, ob der gemäß Element E24 gelesene Barcode mit den gemäß Element E23 geladenen Daten bzw. Informationen übereinstimmt bzw. zu diesen Daten bzw. Informationen passt. Falls nein, wird eine Fehlerreaktion E26 eingeleitet, z.B. das Anzeigen einer Fehlernachricht, und es wird z.B. wieder zu Element E20 übergegangen. Falls ja, wird zu Element E30 verzweigt, das ein Ende des Einlege-Vorgangs symbolisiert.

Bei weiteren beispielhaften Ausführungsformen kann, z.B. ergänzend zu Element E26 in einem Fehlerfall ein Digitalbild DB oder Video von wenigstens einem Teil des eingelegten Blisterbeutelschlauchs 200, z.B. wenigstens von dem ersten Blisterbeutel 200-1 erstellt werden und, z.B. zusammen mit bzw. als Teil einer Fehlernachricht z.B. an das Cloud-System CS gesendet werden, beispielsweise zur Bearbeitung durch eine hierfür vorgesehene Organisationseinheit.

Sofern das Ermitteln E22 ergibt, dass die hochgeladenen Daten (z.B. in Form der zweiten Informationen bzw. der Liste LIST-TT) keine einzelne Blisterbeutel des Blisterbeutelschlauchs 200 identifizierende Daten (z.B. Blisterbeutel-IDs, z.B. "pouch IDs") aufweisen, wird zu Element E27 verzweigt, das das Anzeigen des Medikationsplans symbolisiert.

Element E28 symbolisiert eine Aufforderung, beispielsweise an den Benutzer bzw. das Pflegepersonal, eine Identifikation BD-ID-n des letzten Blisterbeutels 200-n des eingelegten Blisterbeutelschlauchs 200 einzugeben, und, optional, das Empfangen einer entsprechenden Eingabe des Benutzers bzw. Pflegepersonals, der bzw. das die angeforderte Identifikation BD-ID-n beispielsweise manuell bzw. mit Unterstützung eines Geräts, z.B. Mobilgeräts 10, ermittelt und eingibt.

Element E29 symbolisiert ein Ermitteln, ob die angeforderte und eingegebene Identifikation BD-ID-n mit den empfangenen, z.B. zuvor hochgeladenen Daten übereinstimmt bzw. dazu passt. Falls ja, wird zu Element E30 verzweigt. Falls nein, wird eine weitere Fehlerreaktion E31, z.B. unter Ausgabe einer Fehlermeldung, eingeleitet, auf die beispielsweise eine weitergehende Prüfung des Blisterbeutelschlauchs 200, beispielsweise durch manuelle Inspektion durch das Pflegepersonal, folgen kann. Nach der Fehlerreaktion E31 wird beispielsweise wieder zu Element E20 verzweigt.

Element E32 symbolisiert einen Prozess für das Einlegen einer Blisterbeutelrolle gemäß weiteren Ausführungsformen, für den beispielsweise keine, z.B. zuvor manuell ermittelten bzw. hochgeladenen bzw. bereitgestellten Daten verwendet werden. Der Prozess E32 kann beispielsweise dann ausgeführt werden, wenn bereits Daten bzw. zweite Informationen I-2 zu dem einzulegenden Blisterbeutelschlauch 200 vorhanden sind, beispielsweise durch Bereitstellung seitens des Blistercenters BC. Element E33 symbolisiert ein Ende des Ablaufs gemäß weiteren beispielhaften Ausführungsformen.

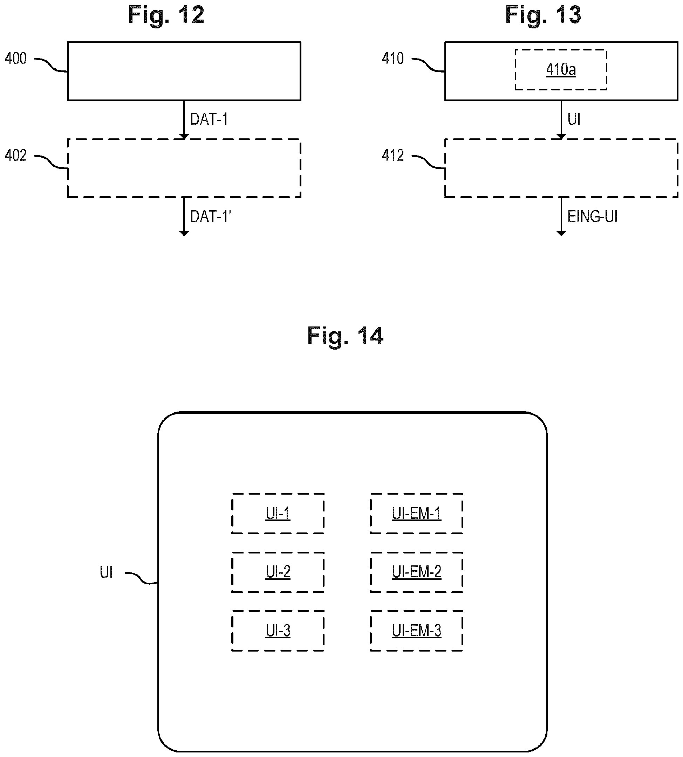

Weitere beispielhafte Ausführungsformen, Fig. 12, beziehen sich auf ein Verfahren, beispielsweise ein computerimplementiertes Verfahren, zum Verarbeiten von mit wenigstens einer Medikamentenausgabevorrichtung 10 (Fig. 3), beispielsweise zur Ausgabe von in Blisterbeuteln bzw. in einem Blisterbeutelschlauch 200 angeordneten Medikamenten, assoziierten Daten, aufweisend: Empfangen 400 von mit der wenigstens einen Medikamentenausgabevorrichtung 100 assoziierten ersten Daten DAT-1, und, optional, Senden 402 wenigstens eines Teils DAT-1' der ersten Daten DAT-1 an die wenigstens eine Medikamentenausgabevorrichtung 100, wobei beispielsweise die ersten Daten DAT-1 wenigstens eines der folgenden Elemente aufweisen: a) erste Informationen I-1, die zumindest Teile eines mit wenigstens einem Medikament assoziierten Medikationsplans charakterisieren, b) zweite Informationen I-2 für eine Steuerung der wenigstens einen Medikamentenausgabevorrichtung 100 (z.B. in Form der Liste LIST-TT), c) dritte Informationen I-3, die von wenigstens einem Teil 200-T eines Blisterbeutelschlauchs 200 einlesbar sind.

Das Verfahren gemäß Fig. 12 kann bei weiteren beispielhaften Ausführungsformen z.B. in einem Cloud-System CS, z.B. wenigstens einer Komponente C-S, E-S des Cloud-System CS, implementiert sein.

Bei weiteren beispielhaften Ausführungsformen, Fig. 13, ist vorgesehen, dass das Verfahren aufweist: Bereitstellen 310 einer, beispielsweise grafischen, Benutzeroberfläche UI, s. z.B. auch Fig. 14, zur Eingabe wenigstens eines Teils der ersten Daten DAT-1, und, optional, Empfangen 412 von mit der Benutzeroberfläche UI assoziierten Benutzereingaben EING-UI.

Das Verfahren gemäß Fig. 13 kann bei weiteren beispielhaften Ausführungsformen z.B. in einem Cloud-System CS, z.B. wenigstens einer Komponente C-S, E-S des Cloud-System CS, implementiert sein, und/oder in einer Medikamentenausgabevorrichtung 100 und/oder in einem PC und/oder einem Mobilgerät.

Bei weiteren beispielhaften Ausführungsformen, Fig. 13, 14, erfolgt das Bereitstellen 410 der Benutzeroberfläche UI beispielsweise durch ein Bereitstellen 410a von entsprechenden Benutzeroberflächenelementen, beispielsweise mittels des Hypertext Transfer Protocol (HTTP), beispielsweise über eine (virtuelle) private Netzwerkverbindung.

Bei weiteren beispielhaften Ausführungsformen, Fig. 14, ist vorgesehen, dass die Benutzeroberfläche UI wenigstens eines der folgenden Elemente aufweist: a) eine Übersicht UI-1 über wenigstens einen, beispielsweise mittels eines Cloud-Systems CS verwaltbaren, Medikationsplan, b) eine Übersicht UI-2 über, beispielsweise mittels eines Cloud-Systems CS verwaltbarer, Benutzer, beispielsweise Patienten, c) eine benutzerindividuelle, beispielsweise patientenindividuelle, Übersicht UI-3 über wenigstens einen, beispielsweise mittels eines Cloud-Systems CS verwaltbaren, Medikationsplan, d) eine Eingabemaske UI-EM-1 zur Erstellung eines Medikationsplans für einen Benutzer, e) eine Eingabemaske UI-EM-2 zur Eingabe einer Identifikation 200-ID eines Blisterbeutelschlauchs 200, f) eine Eingabemaske UI-EM-3 zur Eingabe von Einnahmezeitpunkten.

Weitere beispielhafte Ausführungsformen, Fig. 15, beziehen sich auf eine Vorrichtung 500 zur Ausführung des Verfahrens gemäß den Ausführungsformen. Die Vorrichtung 500 weist wenigstens eine Recheneinrichtung 502 ("Computer") auf, wenigstens eine der Recheneinrichtung 502 zugeordnete Speichereinrichtung 504 zur zumindest zeitweisen Speicherung eines Computerprogramms PRG zur Ausführung wenigstens mancher Aspekte gemäß den Ausführungsformen und/oder von Daten DAT, beispielsweise charakterisierend wenigstens eines der folgenden Elemente: I-1, I-2, I-3, DAT-1.

Bei weiteren beispielhaften Ausführungsformen weist die Recheneinrichtung 502 wenigstens eines der folgenden Elemente auf: einen Mikroprozessor, einen Mikrocontroller, einen digitalen Signalprozessor (DSP), einen programmierbaren Logikbaustein (z.B. FPGA, field programmable gate array), einen ASIC (anwendungsspezifischen integrierten Schaltkreis). Kombinationen hieraus sind bei weiteren bevorzugten Ausführungsformen auch denkbar.

Bei weiteren beispielhaften Ausführungsformen weist die Speichereinrichtung 504 wenigstens eines der folgenden Elemente auf: einen flüchtigen Speicher 504a, beispielsweise Arbeitsspeicher (RAM), einen nichtflüchtigen Speicher 504b, beispielsweise Flash-EEPROM.

Bei weiteren beispielhaften Ausführungsformen weist die Vorrichtung 500 eine optionale Datenschnittstelle 506 auf, über die Daten mit wenigstens einer weiteren Einheit austauschbar sind.

Weitere beispielhafte Ausführungsformen beziehen sich auf ein computerlesbares Speichermedium SM, umfassend Befehle PRG, die bei der Ausführung durch einen Computer 502 diesen veranlassen, das Verfahren gemäß den Ausführungsformen auszuführen.

Weitere beispielhafte Ausführungsformen beziehen sich auf ein Computerprogramm PRG, umfassend Befehle, die bei der Ausführung des Programms PRG durch einen Computer 502 diesen veranlassen, das Verfahren gemäß den Ausführungsformen auszuführen.

Weitere beispielhafte Ausführungsformen beziehen sich auf ein Datenträgersignal DCS, das das Computerprogramm PRG gemäß den Ausführungsformen überträgt und/oder charakterisiert. Beispielsweise ist das Datenträgersignal DCS übertragbar über die optionale Datenschnittstelle 506.

Weitere beispielhafte Ausführungsformen, Fig. 16, beziehen sich auf ein Mobilgerät 10, beispielsweise Smartphone, aufweisend eine Vorrichtung 500 gemäß den Ausführungsformen.

Weitere beispielhafte Ausführungsformen beziehen sich auf eine Medikamentenausgabevorrichtung 100 (Fig. 3), beispielsweise zur Ausgabe von in Blisterbeuteln bzw. in einem Blisterbeutelschlauch angeordneten Medikamenten, aufweisend eine Vorrichtung 500 gemäß den Ausführungsformen. Beispielsweise kann eine Funktionalität der Vorrichtung 500 in der Steuerungseinrichtung 101 integriert sein.

Weitere beispielhafte Ausführungsformen, Fig. 17, beziehen sich auf eine Verwendung 600 des Verfahrens gemäß den Ausführungsformen und/oder der Vorrichtung 500 gemäß den Ausführungsformen und/oder des Mobilgeräts 10 gemäß den Ausführungsformen und/oder der Medikamentenausgabevorrichtung 100 gemäß den Ausführungsformen und/oder des computerlesbaren Speichermediums SM gemäß den Ausführungsformen und/oder des Computerprogramms PRG gemäß den Ausführungsformen und/oder des Datenträgersignals DCS gemäß den Ausführungsformen für wenigstens eines der folgenden Elemente: a) Verarbeiten 601 von mit wenigstens einer Medikamentenausgabevorrichtung 100 assoziierten Daten, b) Bilden 602 von Informationen für eine Steuerung wenigstens einer Medikamentenausgabevorrichtung, c) Übermitteln, beispielsweise Hochladen, 603 von Informationen für eine Steuerung wenigstens einer Medikamentenausgabevorrichtung 100, beispielsweise zu einem Cloud-System CS, d) Bilden 604 und/oder Speichern 605 von Vorlagen für wenigstens einen Medikationsplan, e) Abgleichen 606 von Daten, z.B. für einen Betrieb wenigstens einer Medikamentenausgabevorrichtung, f) Modifizieren 607 von Daten, z.B. für einen Betrieb wenigstens einer Medikamentenausgabevorrichtung, g) Ermöglichen 608 einer, beispielsweise automatischen, Überprüfung wenigstens eines Blisterbeutels, beispielsweise eines letzten Blisterbeutels, eines Blisterbeutelschlauchs, h) Nutzen 609 von wenigstens einem Mobilgerät und/oder von wenigstens einer Medikamentenausgabevorrichtung und/oder von wenigstens einer weiteren Vorrichtung wie z.B. einer Inspektionseinrichtung einer Fertigungseinrichtung für Blisterbeutelschläuche zum Einlesen von Informationen von wenigstens einem Teil eines Blisterbeutelschlauchs, i) Zuordnen 610 von Elementen eines Medikationsplans zu entsprechenden mit einer Produktion eines Blisterbeutelschlauchs assoziierten Elementen.

Nachfolgend sind beispielhaft weitere Aspekte und/oder Ausführungsformen beschrieben, die z.B. gemäß weiteren beispielhaften Ausführungsformen jeweils einzeln für sich und/oder in Kombination miteinander mit wenigstens einem der vorstehend beispielhaft beschriebenen Aspekte kombinierbar sind.

Bei weiteren beispielhaften Ausführungsformen können manche, beispielsweise mit der Medikamentenausgabevorrichtung 100 assoziierte, Daten als vor einer Herstellung eines Blisterbeutelschlauchs 200 bestehende Daten oder "Soll-Daten" bezeichnet werden, z.B. Daten eines Medikationsplans, der zur z.B. Herstellung des Blisterbeutelschlauchs 200 verwendet wird (z.B. charakterisierend eine Menge von Elementen, z.B. bestehend aus Zeitraum, Datum/Uhrzeit des Einnahmezeitpunkts der Medikamente, Patientenname). Optional können auch Daten wenigstens eines Medikationsplans einer vorangehenden Periode, beispielsweise mit einem früher hergestellten bzw. verwendeten Blisterbeutelschlauchs 200 assoziierte Daten, als (Teil der) Soll-Daten angesehen werden.

Bei weiteren beispielhaften Ausführungsformen können manche, beispielsweise mit der Medikamentenausgabevorrichtung 100 assoziierte, Daten als nach einer Herstellung des Blisterbeutelschlauchs 200 vorliegende Daten oder "Ist-Daten" bezeichnet werden, z.B. produktionsbezogene Informationen (Identifikationen von Blisterbeuteln, z.B. "Beutel IDs"), Daten aus dem Medikationsplan, die mit den Daten vor der Herstellung des Blisterbeutelschlauchs 200 ("Blisterherstellung") übereinstimmen müssen (z.B. Datum/Uhrzeit des Einnahmezeitpunkts, Patientenname).

Bei weiteren beispielhaften Ausführungsformen können die zweiten Informationen I-2, z.B. automatisch, z.B. unter Verwendung einer bzw. in der Medikamentenausgabevorrichtung 100, erzeugt werden, beispielsweise mittels wenigstens einem der folgenden Aspekte: Blisterbeutelschlauch 200 einlegen und einmal, z.B. komplett, durchlaufen lassen, z.B. mittels Fördern 314 (Fig. 2), dabei z.B. Bilder DB der Blisterbeutel aufnehmen und, z.B. lokal, z.B. mit einer Software PRG für den Computer 502 (Fig. 15), ggf. einer Zeichenerkennung, z.B. OCR, unterwerfen, und auf dieser Basis z.B. eine Liste LIST-TT erzeugen und ggf. in die Cloud, z.B. das Cloud-System CS, hochladen.

Bei weiteren beispielhaften Ausführungsformen können die zweiten Informationen I-2, z.B. zumindest teilweise manuell, erzeugt werden, beispielsweise mittels wenigstens einem der folgenden Aspekte: Einnahmezeitpunkte ("taking time(s)", TT) und/oder Start Barcodenummer (z.B. charakterisierend eine Identifikation eines ersten Blisterbeutels) und Barcode des letzten Beutels (z.B. charakterisierend eine Identifikation eines letzten Blisterbeutels) eines Blisterbeutelschlauchs 200 in wenigstens einer Eingabemaske eintragen, beispielsweise über das Netzwerk NW, beispielsweise von einem Personal Computer PC aus, woraus z.B. mittels eines Cloud-Servers C-S die Liste LIST-TT bildbar ist.

Bei weiteren beispielhaften Ausführungsformen können die zweiten Informationen I-2, z.B. zumindest teilweise manuell, erzeugt werden, beispielsweise mittels wenigstens einem der folgenden Aspekte: Mit einem Mobilgerät 10 (Fig. 16), beispielsweise Smartphone o.ä. Gerät, z.B. mit Kamera 12: (z.B. alle) Blisterbeutel fotografieren, Bilder in die Cloud, z.B. zu dem Cloud-System CS hochladen, und z.B. mit einer Erkennung, z.B. Zeichenerkennung, z.B. OCR, in der Cloud, z.B. in dem Cloud-Server C-S, z.B. als Liste LIST-TT erstellen.

Bei weiteren beispielhaften Ausführungsformen können die zweiten Informationen I-2, z.B. zumindest teilweise manuell, erzeugt werden, beispielsweise mittels wenigstens einem der folgenden Aspekte: Mit einem externen Gerät (z.B. extern bezüglich der Medikamentenausgabevorrichtung 100), z.B. mit einer Inspektionseinrichtung INSP (Fig. 3) eines Blistercenters BC, und/oder bei einem Pflegedienst mehrere, beispielsweise alle, Blisterbeutel fotografieren, basierend auf den dabei erhaltenen Bilder, z.B. Digitalbildern, ggf. eine Zeichenerkennung, z.B. OCR, ausführen, z.B. in der Cloud, und z.B. die basierend auf der Zeichenerkennung die Liste LIST-TT bereitstellen.

Bei weiteren beispielhaften Ausführungsformen können ein oder mehrere der folgenden Aspekte bzw. Ausführungsformen vorgesehen werden:
a) manuelle, z.B. online-, Eingabe (z.B. über ein Web- bzw. HTML-Formular oder dergleichen) von Daten, z.B. der Eck-Daten aus dem Medikationsplan, z.B. in wenigstens einer Cloud-Eingabe-Maske UI-EM-1, UI-EM-2, ... (Fig. 14), z.B. mit Einnahme-Zeit und/oder Tag und/oder Woche, und z.B. mit einer Erzeugung einer Daten-Tabelle, z.B. vergleichbar mit einer von dem Blistercenter BC zur Verfügung gestellten Tabelle. Bei weiteren beispielhaften Ausführungsformen kann die Medikamentenausgabevorrichtung 100 keinen Unterschied feststellen zu einer Situation ohne die manuelle online-Eingabe, also z.B. zu einem Betrieb mit einer direkt von dem Blistercenter BC (ggf. über das Netzwerk NW) erhaltenen Liste LIST-TT.
b) Medikations-Ausgabe-Daten, z.B. die zweiten Informationen I-2, durch einen z.B. kompletten und z.B. direkten Blisterbeutelschlauch-Durchlaufscan und mit Datenerkennung, z.B. per OCR, eines vorliegenden Blisterbeutelschlauchs 200, mit externen Lesegeräten wie z.B. Kamerastativ, Scanvorrichtung, Smartphone, Medikamentenausgabevorrichtung 100, z.B. Dispenser, Blisterinspektor INSP, u.ä., z.B. vor einem Einlegen des Blisterbeutelschlauchs in die Medikamentenausgabevorrichtung 100, z.B. durch eine Pflegekraft, und z.B. mit einer "Überwachung" durch die betreuende Pflegekraft. Bei weiteren beispielhaften Ausführungsformen kann die Medikamentenausgabevorrichtung 100 bei diesem Aspekt keinen Unterschied feststellen zu einem Betrieb mit einer direkt von dem Blistercenter BC (ggf. über das Netzwerk NW) erhaltenen Liste LIST-TT.

Bei weiteren beispielhaften Ausführungsformen können ein oder mehrere der folgenden Aspekte bzw. Ausführungsformen vorgesehen werden, beispielsweise für ein zumindest teilweise manuelles Verarbeiten von mit der Medikamentenausgabevorrichtung 100 assoziierten Daten, beispielsweise für das Bereitstellen der zweiten Informationen I-2:
a) Hochladen von mit Blisterbeutelschläuchen, z.B. Blisterbeutelrollen, assoziierten Daten, z.B. mit oder ohne Rollen-IDs 200-ID, z.B. auf Basis von individuellen Medikationsplänen wenigstens eines Patienten,
b) Speichern von Medikationsplan-Vorlagen, z.B. für ein zukünftiges schnelleres Hochladen bzw. Bereitstellen der zweiten Informationen, z.B. für zukünftige Blisterbeutelschläuche bzw. Blisterbeutelrollen,
c) Anzeigen eines, beispielsweise genauen, Dispensing-Schemas (z.B. Schema, das Ausgabezeiten von in den Blisterbeuteln eines Blisterbeutelschlauchs angeordneten Medikamenten charakterisiert), z.B. basierend auf dem Cloud-System CS, z.B. "in der Cloud", z.B. als Kontrolle, z.B. für Pflegekräfte,
d) Überwachung eines Status von Medikationsplänen, z.B. aufweisend d1) ein Ermitteln, ob noch Daten vorhanden sind für einen Patienten, d2) Ermitteln, ob eine Blisterbeutelrolle ordnungsgemäß eingelegt ist,
e) Abgleich von, z.B. in das Cloud-System CS hochgeladenen, Daten und wenigstens einer einzulegenden Blisterbeutelrolle, z.B. basierend auf einem Einnahmezeitpunkt eines letzten Blisterbeutels auf der Blisterbeutelrolle,
f) Definieren von, z.B. verteilten, Aufgaben, z.B. in der Cloud bzw. in dem Cloud-System CS und an einem Gerät, z.B. der Medikamentenausgabevorrichtung 100 (z.B. bezüglich des Medikationsplans, Kontrolle/Vervollständigung des Medikationsplans, z.B. beim Einlegen einer neuen Blisterbeutelrolle. Z.B. kann eine Pflegekraft vor Ort, d.h. an bzw. mittels der Medikamentenausgabevorrichtung 100 die zweiten Informationen, beispielsweise die Liste LIST-TT bzw. einen entsprechenden Plan, z.B. Zeitplan, anpassen und/oder ergänzen,
g) automatisierte (z.B. ohne menschliche Interaktion) Erkennung und/oder Kontrolle eines letzten Blisterbeutels, z.B. durch die Medikamentenausgabevorrichtung 100,
h) Blisterbeutelkontrolle, beispielsweise vollständige Blisterbeutelkontrolle, z.B. einer ganzen Blisterbeutelrolle, z.B. durch Prüfen aller Blisterbeutel, z.B. beim Einlegen in die Medikamentenausgabevorrichtung 100,
i) weitere Abläufe z.B. unter Verwendung von Zeichenerkennung, z.B. OCR, und/oder Code-Scannen, z.B. steuerbar durch Smartphone-App APP (Fig. 16), ausführbar z.B. auch auf anderen Geräten wie z.B. der Medikamentenausgabevorrichtung 100, einem Personal Computer PC (Fig. 3), Tablet-Computer, usw..

Bei weiteren beispielhaften Ausführungsformen kann z.B. von einem Therapieplan (vorher, Soll) und z.B. einer Menge von Therapiemaßnahmen (nachher, Ist) gesprochen werden, deren Daten unter Verwendung des Prinzips gemäß den Ausführungsformen miteinander verbunden werden.

Bei weiteren beispielhaften Ausführungsformen wird wenigstens einem, z.B. jedem, Element des Medikationsplans (Soll) wenigstens ein entsprechendes Element der produktionsbezogenen Daten (Ist) zugeordnet.

Bei weiteren beispielhaften Ausführungsformen kann ein Abgleich von Daten, z.B. von mit der Medikamentenausgabevorrichtung 100 assoziierten Daten, durch wenigstens einen der folgenden Aspekte oder durch beliebige Kombinationen daraus erfolgen:
A) Vergleich und Zuordnung von einzelnen Elementpaaren und, optional, Fortschreibung der folgenden oder dazwischenliegenden Elemente.
   A1) Z.B. Vergleich und Zuordnung einer Identifikation, z.B. "Pouch ID", z.B. "Blisterbeutel-ID", zu einem ersten Blisterbeutel 200-1, und, basierend darauf, Zuordnung einer Identifikation, z.B. Pouch ID(s), zu den auf den ersten Blisterbeutel folgenden Beuteln. In diesem Fall ist ein zweiter Faktor für den Datenabgleich z.B. ein Vergleich eines auf einem Blisterbeutel angeordneten, beispielsweise aufgedruckten, Einnahmezeitpunkts, z.B. des letzten Blisterbeutels, mit einem letzten Einnahmezeitpunkt des Medikationsplans.
   A2) Z.B. Vergleich und Zuordnung einer Pouch ID zu einem ersten Blisterbeutel 200-1 und zu einem letzten Blisterbeutel 200-n, Zuordnung der dazwischenliegenden Pouch IDs zu den dazwischenliegenden Blisterbeuteln, optional Kontrolle über eine Anzahl der Blisterbeutel.
   A3) Bei einer Fortschreibung z.B. von Pouch IDs können z.B. bestimmte, z.B. vorgebbare, Schemata (z.B. nächste Pouch ID jeweils +1, -1, +10, ...) zugrunde gelegt werden. Bei weiteren beispielhaften Ausführungsformen können die Schemata z.B. einzelnen Herstellern der Therapiemaßnahmen bzw. Blisterbeutelschläuche, z.B. Blistercentern, zugeordnet werden.
B) Vollständiger Vergleich aller Elemente, z.B. werden bei allen Blisterbeuteln eines Blisterbeutelschlauchs 200, z.B. automatisch, die Ist-Daten gelesen und mit den Soll-Daten verglichen, und es werden z.B. die zusätzlichen Ist-Daten dem Soll-Datensatz zugeordnet.
C) Vergleich einzelner Elementpaare, und, z.B. bei Implausibilitäten, z.B. Vergleich weiterer/aller Elementpaare. Wenn z.B. eine Erhöhung der Blisterbeutel-IDs nicht zu einer Anzahl der Blisterbeutel passt oder wenn eine einzelne Blisterbeutel-ID nicht einem erwarteten Schema entspricht, kann der betreffende Blisterbeutel z.B. gesondert verglichen werden.

Bei weiteren beispielhaften Ausführungsformen kann ein Abgleich bzw. Vergleich z.B. erfolgen durch wenigstens eines der folgenden Elemente:
a) Anzeige und Bestätigung durch Anwender, z.B. zeigt die Medikamentenausgabevorrichtung 100 einen Namen eines Patienten an, und ein Anwender liest den Namen von einem Blisterbeutel bzw. der Blisterbeutelrolle ab und bestätigt nach dem Ablesen den angezeigten Namen, z.B. durch eine Eingabe unter Verwendung z.B. eines Benutzeroberflächenelements. Beispielsweise kann die Medikamentenausgabevorrichtung 100 alternativ oder ergänzend einen letzten Einnahmezeitpunkt eines Medikaments anzeigen, und ein Anwender kann, z.B. nach einem Ablesen entsprechender Zeitinformationen von der Blisterbeutelrolle, den angezeigten Einnahmezeitpunkt bestätigen.
b) Bei weiteren beispielhaften Ausführungsformen ist ein z.B. zumindest teilweise automatisiertes Lesen der Ist-Daten und ein Vergleich mit den Soll-Daten vorgesehen. Das z.B. automatische Lesen kann z.B. erfolgen durch Lesen von 1D / 2D-Barcodes, RFID, NFC, oder OCR, z.B. mittels der Leseeinrichtung 102 und/oder 12. Das Lesen kann z.B. auf der Medikamentenausgabevorrichtung 100 selbst und/oder mit einem Mobiltelefon 10 oder einem sonstigen externen Gerät erfolgen.

Bei weiteren beispielhaften Ausführungsformen wird z.B. ein Barcode und ein z. B. lesbaren Text aufweisender Aufdruck (z.B. per OCR) z.B. eines letzten Blisterbeutels gelesen, beispielsweise unter Verwendung des Smartphone 10 (Fig. 16), beispielsweise unter Steuerung der Applikation APP. Danach wird z.B. beim Einlegen einer neuen Blisterbeutelrolle der Barcode des ersten Blisterbeutels gelesen und mit den Soll-Daten verglichen.

Bei weiteren beispielhaften Ausführungsformen liest die Medikamentenausgabevorrichtung 100 und/oder das Smartphone 10, z.B. mittels der Applikation APP, Informationen mehrerer Blisterbeutel nacheinander ein, ggf. unter Anwendung von Zeichenerkennung, z.B. OCR, ermittelt auf dieser Basis z.B. einen Beutelaufdruck des jeweiligen Blisterbeutels und vergleicht den ermittelten Beutelaufdruck mit den Soll-Daten.

Bei weiteren beispielhaften Ausführungsformen erfolgt eine Eingabe der Ist-Daten z.B. durch einen Anwender bzw. Benutzer, und sodann kann ein Vergleich der eingegebenen Ist-Daten mit Soll-Daten ausgeführt werden. Z.B. kann ein Anwender Datum und Uhrzeit eines letzten Einnahmezeitpunkts der betrachteten Blisterbeutelrolle eingeben, und diese Daten werden bei weiteren beispielhaften Ausführungsformen abgeglichen mit den Soll-daten, beispielsweise durch die Vorrichtung 500 bzw. ein ihre Funktionalität enthaltendes Zielsystem 100, E-S, C-S, PC.

Das Prinzip gemäß den Ausführungsformen ermöglicht vorteilhaft eine Bereitstellung der zweiten Informationen I-2, z.B. für einen Betrieb der wenigstens einen Medikamentenausgabevorrichtung 100. Zudem wird eine Multi-Faktor Überprüfung von mit der Medikamentenausgabevorrichtung 100 assoziierten Daten ermöglicht, die eine sichere Medikamentenausgabe durch die Medikamentenausgabevorrichtung 100 ermöglicht. Bei zumindest manchen beispielhaften Ausführungsformen kann eine gesteigerte Sicherheit bezüglich der Medikamentenausgabe durch die Medikamentenausgabevorrichtung 100 bereits durch vergleichsweise wenig aufwendige bzw. wenig umfassende z.B. manuelle Dateneingaben erreicht werden.

## Patentansprüche

1. Verfahren, beispielsweise computerimplementiertes Verfahren, zum Verarbeiten von mit wenigstens einer Medikamentenausgabevorrichtung (100), beispielsweise zur Ausgabe von in Blisterbeuteln bzw. in einem Blisterbeutelschlauch (200) angeordneten Medikamenten, assoziierten Daten, aufweisend: Ermitteln (300) von ersten Informationen (I-1), die zumindest Teile eines mit wenigstens einem Medikament assoziierten Medikationsplans charakterisieren, Bilden (302) von zweiten Informationen (I-2) für eine Steuerung der wenigstens einen Medikamentenausgabevorrichtung (100) basierend wenigstens auf den ersten Informationen (I-1), und, optional, Bereitstellen (304) der zweiten Informationen (I-2), beispielsweise für eine Nutzung (306) der zweiten Informationen (I-2) durch die wenigstens eine Medikamentenausgabevorrichtung (100).

2. Verfahren nach Anspruch 1, aufweisend: Einlesen (310) von dritten Informationen (I-3) von wenigstens einem Teil (200-T) eines Blisterbeutelschlauchs (200), wobei der wenigstens eine Teil (200-T) des Blisterbeutelschlauchs (200) beispielsweise wenigstens zwei, beispielsweise zueinander benachbarte, Blisterbeutel des Blisterbeutelschlauchs (200) aufweist, Ermitteln (312) der ersten Informationen (I-1) basierend wenigstens auf den dritten Informationen (I-3).

3. Verfahren nach Anspruch 2, wobei für das Einlesen (310) der dritten Informationen (I-3) wenigstens eines der folgenden Elemente verwendet wird: a) eine, beispielsweise lokale, Leseeinrichtung (102, 310a) der Medikamentenausgabevorrichtung (100), b) wenigstens eine weitere Leseeinrichtung (310b), beispielsweise eine Leseeinrichtung (12) eines Mobilgeräts (10), beispielsweise eines Smartphones oder Tablet-Computers, c) ein manueller Einlesevorgang (310c), beispielsweise durch einen Benutzer (BEN), beispielsweise ein Pflegepersonal (PFP).

4. Verfahren nach wenigstens einem der Ansprüche 2 bis 3, wobei für das Ermitteln (312) der ersten Informationen (I-1) wenigstens eines der folgenden Elemente verwendet wird: a) eine, beispielsweise lokale, Ermittlungseinrichtung (104; 312a) der Medikamentenausgabevorrichtung (100), b) wenigstens eine weitere Ermittlungseinrichtung (312b), beispielsweise eine entfernt zu der Medikamentenausgabevorrichtung (100) angeordnete Ermittlungseinrichtung, beispielsweise eine Edge- und/oder Cloud-basierte Ermittlungseinrichtung, c) ein manueller Ermittlungsvorgang (312c), beispielsweise durch einen Benutzer (BEN), beispielsweise ein Pflegepersonal (PFP), wobei beispielsweise das Ermitteln (312; 312a, 312b) das Ausführen einer Zeichenerkennung, beispielsweise optischen Zeichenerkennung, beispielsweise OCR, aufweist.

5. Verfahren nach wenigstens einem der vorstehenden Ansprüche, aufweisend: Fördern (314) wenigstens eines Teils (200-T) eines bzw. des Blisterbeutelschlauchs (200), wobei der wenigstens eine Teil (200-T) des Blisterbeutelschlauchs (200) beispielsweise wenigstens zwei benachbarte Blisterbeutel des Blisterbeutelschlauchs (200) aufweist, wobei beispielsweise das Fördern (314) des wenigstens einen Teils (200-T) eines bzw. des Blisterbeutelschlauchs (200) wenigstens eines der folgenden Elemente aufweist: a) Fördern (314a) des wenigstens einen Teils (200-T) des Blisterbeutelschlauchs (200) mittels einer Fördereinrichtung (106) der Medikamentenausgabevorrichtung (100), b) manuelles Fördern (314b), beispielsweise durch einen Benutzer (BEN), beispielsweise ein Pflegepersonal (PFP).

6. Verfahren nach wenigstens einem der vorstehenden Ansprüche, wobei das Bilden (302) der zweiten Informationen (I-2) aufweist: Bilden (302a) einer Liste (LIST-TT), die Einnahmezeitpunkte des wenigstens einen Medikaments charakterisiert.

7. Verfahren nach wenigstens einem der vorstehenden Ansprüche, wobei das Bereitstellen (304) der zweiten Informationen (I-2) wenigstens eines der folgenden Elemente aufweist: a) Speichern (304a) der zweiten Informationen (I-2), beispielsweise in Form einer Liste (LIST-TT), in einer Speichereinrichtung (105) der Medikamentenausgabevorrichtung (100), b) Senden (304b) der zweiten Informationen (I-2), beispielsweise über ein, z.B. zumindest bereichsweise öffentliches oder privates oder virtuelles privates, Netzwerk (NW), beispielsweise an eine weitere Einheit, beispielsweise einen Edge-Server (E-S) und/oder einen Cloud-Server (C-S).

8. Verfahren nach wenigstens einem der vorstehenden Ansprüche, aufweisend wenigstens eines der folgenden Elemente: a) Einlegen (320) eines Blisterbeutelschlauchs (200) in eine bzw. die Medikamentenausgabevorrichtung (100), b) Fördern (322) wenigstens eines Teils (200-T) des Blisterbeutelschlauchs (200), beispielsweise mittels einer bzw. der Fördereinrichtung (106) der Medikamentenausgabevorrichtung (100), wobei der wenigstens eine Teil (200-T) des Blisterbeutelschlauchs (200) beispielsweise wenigstens zwei benachbarte Blisterbeutel des Blisterbeutelschlauchs (200) aufweist, relativ zu einer Leseeinrichtung (102) der Medikamentenausgabevorrichtung (100), c) Einlesen (324) von dritten Informationen (I-3) von wenigstens einem Teil (200-T) des Blisterbeutelschlauchs (200), beispielsweise mittels der Leseeinrichtung (102), d) optional Ermitteln (325) der zweiten Informationen (I-2) basierend wenigstens auf den dritten Informationen (I-3), e) Senden der zweiten Informationen (I-2) und/oder der dritten Informationen (I-3) an wenigstens eine weitere Einheit, beispielsweise an einen Edge-Server (E-S) und/oder einen Cloud-Server (C-S), beispielsweise über ein, z.B. zumindest bereichsweise öffentliches oder privates oder virtuelles privates, Netzwerk (NW).

9. Verfahren nach wenigstens einem der vorstehenden Ansprüche, aufweisend wenigstens eines der folgenden Elemente: a) Ermitteln (330) einer ersten Identifikation (BB-ID-1), die mit einem ersten Blisterbeutel (200-1) eines bzw. des Blisterbeutelschlauchs (200) assoziiert ist, b) Ermitteln (332) einer zweiten Identifikation (BB-ID-n), die mit einem weiteren, beispielsweise letzten, Blisterbeutel (200-n) des Blisterbeutelschlauchs (200) assoziiert ist, c) Ermitteln (334) wenigstens einer weiteren Information (I-w1) des Blisterbeutelschlauchs (200), wobei beispielsweise die wenigstens eine weitere Information (I-w1) einen mit wenigstens einem Blisterbeutel des Blisterbeutelschlauchs (200) assoziierten Einnahmezeitpunkt und/oder eine Identifikation (200-ID) des Blisterbeutelschlauchs (200) charakterisiert, d) Bilden (336) der zweiten Informationen (I-2), beispielsweise in Form einer Liste (LIST-TT), basierend auf wenigstens einem der folgenden Elemente: d1) der ersten Identifikation (BB-ID-1), d2) der zweiten Identifikation (BB-ID-2), d3) der wenigstens einen weiteren Information (I-w1).

10. Verfahren nach wenigstens einem der vorstehenden Ansprüche, aufweisend wenigstens eines der folgenden Elemente: a) Erstellen (340) wenigstens eines Digitalbilds (DB) und/oder Videos (VID), wenigstens mancher, beispielsweise aller, Blisterbeutel (200-1, ..., 200-n) des Blisterbeutelschlauchs (200), b) optional Senden (342) des wenigstens einen Digitalbilds (DB) und/oder Videos (VID) an wenigstens eine weitere Einheit, beispielsweise an einen Edge-Server (E-S) und/oder einen Cloud-Server (C-S), beispielsweise über ein, z.B. zumindest bereichsweise öffentliches oder privates oder virtuelles privates, Netzwerk (NW), c) optional, Ermitteln (344) der zweiten Informationen (I-2) basierend auf dem wenigstens einen Digitalbild (DB) und/oder Video (VID), d) Senden (346) der zweiten Informationen (I-2) an wenigstens eine weitere Einheit, beispielsweise an einen Edge-Server (ES) und/oder einen Cloud-Server (C-S), beispielsweise über ein, z.B. zumindest bereichsweise öffentliches oder privates oder virtuelles privates, Netzwerk (NW),
wobei beispielsweise für das Erstellen (340) des wenigstens einen Digitalbilds (DB) und/oder Videos (VID) wenigstens eines der folgenden Elemente verwendet wird: a) Digitalkamera, b) Smartphone (10), beispielsweise in das Smartphone integrierte Kamera (12), c) Tablet, beispielsweise in das Tablet integrierte Kamera, d) Mobilgerät, beispielsweise in das Mobilgerät integrierte Kamera, e) Inspektionseinrichtung (INSP), beispielsweise einer Fertigungseinrichtung (BC) für den Blisterbeutelschlauch (200).

11. Verfahren, beispielsweise computerimplementiertes Verfahren, zum Verarbeiten von mit wenigstens einer Medikamentenausgabevorrichtung (100), beispielsweise zur Ausgabe von in Blisterbeuteln bzw. in einem Blisterbeutelschlauch (200) angeordneten Medikamenten, assoziierten Daten, aufweisend: Empfangen (400) von mit der wenigstens einen Medikamentenausgabevorrichtung (100) assoziierten ersten Daten (DAT-1), und, optional, Senden (402) wenigstens eines Teils (DAT-1') der ersten Daten (DAT-1) an die wenigstens eine Medikamentenausgabevorrichtung (100), wobei beispielsweise die ersten Daten (DAT-1) wenigstens eines der folgenden Elemente aufweisen: a) erste Informationen (I-1), die zumindest Teile eines mit wenigstens einem Medikament assoziierten Medikationsplans charakterisieren, b) zweite Informationen (I-2) für eine Steuerung der wenigstens einen Medikamentenausgabevorrichtung (100), c) dritte Informationen (I-3), die von wenigstens einem Teil (200-T) eines Blisterbeutelschlauchs (200) einlesbar sind.

12. Verfahren nach Anspruch 11, aufweisend: Bereitstellen (410) einer, beispielsweise grafischen, Benutzeroberfläche (UI) zur Eingabe wenigstens eines Teils der ersten Daten (DAT-1), und, optional, Empfangen (412) von mit der Benutzeroberfläche (UI) assoziierten Benutzereingaben (EING-UI), wobei beispielsweise die Benutzeroberfläche (UI) wenigstens eines der folgenden Elemente aufweist: a) eine Übersicht (UI-1) über wenigstens einen, beispielsweise mittels eines Cloud-Systems (CS) verwaltbaren, Medikationsplan, b) eine Übersicht (UI-2) über, beispielsweise mittels eines Cloud-Systems (CS) verwaltbarer, Benutzer, c) eine benutzerindividuelle, beispielsweise patientenindividuelle, Übersicht (UI-3) über wenigstens einen, beispielsweise mittels eines Cloud-Systems (CS) verwaltbaren, Medikationsplan, d) eine Eingabemaske (UI-EM-1) zur Erstellung eines Medikationsplans für einen Benutzer, e) eine Eingabemaske (UI-EM-2) zur Eingabe einer Identifikation (200-ID) eines Blisterbeutelschlauchs (200), f) eine Eingabemaske (UI-EM-3) zur Eingabe von Einnahmezeitpunkten.

13. Vorrichtung (500) zur Ausführung des Verfahrens nach wenigstens einem der vorstehenden Ansprüche.

14. Medikamentenausgabevorrichtung (100), beispielsweise zur Ausgabe von in Blisterbeuteln bzw. in einem Blisterbeutelschlauch (200) angeordneten Medikamenten, aufweisend eine Vorrichtung (500) nach Anspruch 13.

15. Verwendung (600) des Verfahrens nach wenigstens einem der Ansprüche 1 bis 12 und/oder der Vorrichtung (500) nach Anspruch 13 und/oder eines wenigstens eine Vorrichtung (500) nach Anspruch 13 aufweisenden Mobilgeräts (10) und/oder der Medikamentenausgabevorrichtung (100) nach Anspruch 14 und/oder eines computerlesbaren Speichermediums (SM) zur Ausführung des Verfahrens nach wenigstens einem der Ansprüche 1 bis 12 und/oder eines Computerprogramms (PRG) zur Ausführung des Verfahrens nach wenigstens einem der Ansprüche 1 bis 12 und/oder eines Datenträgersignals (DCS), das wenigstens ein Computerprogramm (PRG) zur Ausführung des Verfahrens nach wenigstens einem der Ansprüche 1 bis 12 überträgt und/oder charakterisiert, für wenigstens eines der folgenden Elemente: a) Verarbeiten (601) von mit wenigstens einer Medikamentenausgabevorrichtung (100) assoziierten Daten, b) Bilden (602) von Informationen (I-2) für eine Steuerung wenigstens einer Medikamentenausgabevorrichtung (100), c) Übermitteln (603), beispielsweise Hochladen, von Informationen (I-2) für eine Steuerung wenigstens einer Medikamentenausgabevorrichtung (100), beispielsweise zu einem Cloud-System (CS), d) Bilden (604) und/oder Speichern (605) von Vorlagen für wenigstens einen Medikationsplan, e) Abgleichen (606) von Daten, z.B. für einen Betrieb wenigstens einer Medikamentenausgabevorrichtung (100), f) Modifizieren (607) von Daten, z.B. für einen Betrieb wenigstens einer Medikamentenausgabevorrichtung (100), g) Ermöglichen (608) einer, beispielsweise automatischen, Überprüfung wenigstens eines Blisterbeutels, beispielsweise eines letzten Blisterbeutels (200-n), eines Blisterbeutelschlauchs (200), h) Nutzen (609) von wenigstens einem Mobilgerät (10) und/oder von wenigstens einer Medikamentenausgabevorrichtung (100) und/oder von wenigstens einer weiteren Vorrichtung wie z.B. einer Inspektionseinrichtung einer Fertigungseinrichtung für Blisterbeutelschläuche zum Einlesen (310) von Informationen (I-3) von wenigstens einem Teil (200-T) eines Blisterbeutelschlauchs (200), i) Zuordnen (610) von Elementen eines Medikationsplans zu entsprechenden mit einer Produktion eines Blisterbeutelschlauchs (200) assoziierten Elementen.
